# EUROPEAN PATENT SPECIFICATION

(11) **EP 4 125 753 B1**
(45) Date of publication and mention of the grant of the patent: **24.12.2025**
(21) Application number: 21716401.1
(22) Date of filing: 01.04.2021
(51) Int. Cl.: A61F 13/00, A61F 13/02, A61M 1/00, A61M 27/00

(54) **APPARATUSES FOR NEGATIVE PRESSURE WOUND THERAPY**
VORRICHTUNGEN FÜR UNTERDRUCKWUNDTHERAPIE
APPAREILS POUR LE TRAITEMENT DES PLAIES PAR PRESSION NÉGATIVE

(30) Priority: 01.04.2020 GB 202004812
(43) Date of publication of application: 08.02.2023
(73) Proprietor: T.J.Smith and Nephew,Limited, Hull HU3 2BN (GB)
(72) Inventor: EARL, Michael, Hull HU3 2BN (GB); JOHNSON, Danielle Susan, Hull HU3 2BN (GB); KELBIE, William, Inverness, IV3 8QW (GB); LLOYD, David, Hull HU3 2BN (GB); MORTIMER, Samuel John, Hull HU3 2BN (GB); PARRY, Luke Michael, Hull HU3 2BN (GB); STEWARD, Daniel Lee, Hull HU3 2BN (GB); WARD, Rhianna, Hull HU3 2BN (GB); WEEDON, Hannah Bailey, Hull HU3 2BN (GB)
(74) Representative: Appleyard Lees IP LLP
(86) International application number: PCT/EP2021/058696
(87) International publication number: WO 2021/198463

(56) References cited:
- WO-A1-03/057307
- WO-A1-2014/003957
- WO-A2-2013/175306
- GB-A- 2 527 617
- US-A1- 2017 291 020
- US-A1- 2020 069 477

## Description

### BACKGROUND OF THE INVENTION

### Field

Embodiments of the present disclosure relate to methods and apparatuses for dressing and treating a wound with reduced pressure therapy or topical negative pressure (TNP) therapy. In particular, but without limitation, embodiments disclosed herein relate to negative pressure therapy devices, methods for controlling the operation of TNP systems, and methods of using TNP systems.

### Description of the Related Art

Many different types of wound dressings are known for aiding in the healing process of a human or animal. These different types of wound dressings include many different types of materials and layers, for example, gauze, pads, foam pads or multi-layer wound dressings. Topical negative pressure (TNP) therapy, sometimes referred to as vacuum assisted closure, negative pressure wound therapy (NPWT), or reduced pressure wound therapy, is widely recognized as a beneficial mechanism for improving the healing rate of a wound. Such therapy is applicable to a broad range of wounds such as incisional wounds, open wounds and abdominal wounds or the like.

TNP therapy assists in the closure and healing of wounds by reducing tissue oedema, encouraging blood flow, stimulating the formation of granulation tissue, removing excess exudates and may reduce bacterial load and, thus, infection to the wound. Furthermore, TNP therapy permits less outside disturbance of the wound and promotes more rapid healing.

US2017/291020A1 discloses a double disk dermal device for administration of one or more active agents to the skin or mucosa of a host, in particular a patch is described including: a) active reservoir film layer; b) a backing overlay adjacent to the active reservoir film layer extending beyond the perimeter of the reservoir layer in all directions; c) second overlay backing layer with a coating of pressure sensitive adhesive which is adjacent to the first backing overlay extending beyond the perimeter of the first backing overlay in all directions; and d) a removable release liner.

### SUMMARY

Embodiments of the disclosure described herein are directed to apparatuses, systems, devices and methods for use in negative pressure wound therapy. It will be understood by one of skill in the art that application of the materials, devices, methods, and systems described herein are not limited to a particular tissue or a particular injury. The invention is defined by the appended claims, which relate to an apparatus for sealing a wound site. Embodiments, examples or aspects falling outside the scope of the appended claims, for example methods for sealing a wound dressing, are provided for understanding of the invention only.

The invention described herein provides an apparatus for sealing a wound site as defined by claim 1.

The fixation strip may comprise a width and a length. The second adhesive may extend along the entire width of the fixation strip. The first adhesive may extend along a portion of the entire width of the fixation strip.

**In** some embodiments, the at least one protective liner may further comprise a first protective liner and a second protective liner. The first protective liner may be removably adhered to the first adhesive surface of the wound sealing layer and the second protective liner may be removably adhered to the second adhesive surface of the adhering layer. The first protective liner may comprise a release handle that may be configured to remove the first protective liner from the wound sealing layer when actuated. The second protective liner may comprise a release handle that may be configured to remove the second protective liner from the adhering layer when actuated.

**In** some embodiments, the at least one protective liner may further comprise a first outer protective liner, a second outer protective liner and a central protective liner. The first outer protective liner may be removably adhered to an outer edge portion of the first adhesive surface of the wound sealing layer. The second outer protective liner may be removably adhered to a portion of the second adhesive surface of the adhering layer extending outward beyond the wound sealing layer. The central protective liner may be removably adhered to the first adhesive surface of the wound sealing layer between the first and second outer protective liners.

**In** some embodiments, at least one of the first outer protective liner, the second outer protective liner, and the central protective liner may comprise a release handle configured to remove the respective liner from the wound sealing layer and/or the adhering layer when actuated.

In some embodiments, the first outer protective liner, the second outer protective liner, and the central protective liner can each comprise a width that extends a width of the fixation strip to removably adhere to the first adhesive surface of the wound sealing layer and a portion of the second adhesive surface of the adhering layer that extends outward beyond the wound sealing layer.

In some embodiments, the apparatus for sealing a wound site may further comprise a carrier layer that may be removably adhered to a non-adhesive surface of the adhering layer.

In some embodiments, the first adhesive comprises a silicone adhesive configured to adhere to the wound site or to skin surrounding the wound site. In some embodiments, the second adhesive comprises an acrylic adhesive configured to adhere to a wound dressing. In some embodiments, the wound sealing layer comprises a flexible polymer. In some embodiments, the adhering layer comprises a flexible film.

Some embodiments described herein provide a negative wound therapy kit. The negative pressure wound therapy kit may comprise a wound dressing and a fixation strip. The wound dressing may comprise a cover. The fixation strip may comprise a wound sealing layer and an adhering layer. The wound sealing layer may comprise a first adhesive. For example, the wound sealing layer may comprise a first adhesive surface that comprises the first adhesive. The adhering layer may overlay and extend outward beyond the wound sealing layer. The adhering layer may comprise a second adhesive. For example, the adhering layer may comprise a second adhesive surface that comprises the second adhesive. The fixation strip may comprise at least one protective liner removably adhered to the first adhesive surface of the wound sealing layer and/or the second adhesive surface of the adhering layer.

In some embodiments, the at least one protective liner may further comprise a first protective liner and a second protective liner. The first protective liner may be removably adhered to the first adhesive surface of the wound sealing layer. The second protective liner may be removably adhered to the second adhesive surface of the adhering layer.

In some embodiments, a portion of the second adhesive surface of the adhering layer extending outward beyond the wound sealing layer may be configured to adhere to the cover of the wound dressing in use. In some embodiments, the at least one protective liner may further comprise a first outer protective liner, a second outer protective liner and a central protective liner. The first outer protective liner may be removably adhered to an outer edge portion of the first adhesive surface of the wound sealing layer. The second outer protective liner may be removably adhered to the second adhesive surface of the adhering layer extending outward beyond the wound sealing layer. The central protective liner may be removably adhered to the first adhesive surface of the wound sealing layer between the first and second outer protective liners. The outer edge portion of the first adhesive surface of the wound sealing layer may be opposite the portion of the adhering layer extending outward beyond the wound sealing layer.

In some embodiments, the first outer protective liner, the second outer protective liner, and the central protective liner can each comprise a width that extends a width of the fixation strip to removably adhere to the first adhesive surface of the wound sealing layer and a portion of the second adhesive surface of the adhering layer that extends outward beyond the wound sealing layer.

In some embodiments, the fixation strip may further comprise a carrier layer that may be removably adhered to a non-adhesive surface of the adhering layer.

Some of the embodiments described herein provide a method for sealing a wound dressing. The method may comprise positioning a wound dressing over a wound site and a periwound area surrounding the wound site; removing a central protective liner and a first outer protective liner from a first adhesive surface of a wound sealing layer and/or a second adhesive surface of an adhering layer of a fixation strip; positioning the fixation strip over the periwound area and partially over the wound dressing; removing a second outer protective liner from the first adhesive surface of the wound sealing layer and/or the second adhesive surface of the adhering layer of the fixation strip; adhering the adhering layer of the fixation strip to the cover of the wound dressing; positioning an applicator of negative pressure on the cover of the wound dressing; and applying negative pressure to the wound site and the periwound area through the wound dressing for a period of time. The wound dressing may comprise the cover. The fixation strip may comprise the wound sealing layer, the adhering layer, the central protective liner the first outer protective liner, and the second outer protective liner. The wound sealing layer of the fixation strip may comprise the first adhesive surface comprising a first adhesive. The adhering surface may overlay and extend outward beyond the wound sealing layer. The adhering layer of the fixation strip may comprise the second adhesive surface comprising a second adhesive.

In some embodiments, the method disclosed may further comprise cutting the wound dressing along a side portion of the wound dressing prior to positioning the wound dressing on the wound site and the periwound area.

Some of the embodiments described herein provide a method for sealing a wound dressing. The method may comprise cutting a wound dressing along a side portion of the wound dressing; using a release handle of a first protective liner to remove the first protective liner from a first adhesive surface of a wound sealing layer of a fixation strip; positioning the fixation strip over a periwound area surrounding a wound site; inserting the side portion of the wound dressing into the fixation strip between the wound sealing layer and an adhering layer; positioning the wound dressing over the wound site and the periwound area; using a release handle of a second protective liner to remove the second protective liner from a second adhesive surface of the adhering layer; adhering the adhering layer of the fixaton strip to the wound dressing; positioning an applicator of negative pressure on the wound dressing; and applying negative pressure to the wound site and the periwound area through the wound dressing for a period of time. The wound dressing may comprise the cover. The fixation strip may comprise the wound sealing layer, the adhering layer, the first protective liner, and the second protective liner. The wound sealing layer of the fixation strip may comprise the first adhesive surface comprising a first adhesive. The adhering surface may overlay and extend outward beyond the wound sealing layer. The adhering layer of the fixation strip may comprise the second adhesive surface comprising a second adhesive. The first protective liner of the fixation strip may comprise a release handle. The second protective liner of the fixation strip may comprise a release handle.

In some embodiments, the fixation strip may further comprise a carrier layer that may be removably adhered to a non-adhesive surface of the adhering layer. The method disclosed may further comprise removing the carrier layer from the fixation strip prior to applying negative pressure to the wound site and the periwound area.

Some of the embodiments described herein provide an apparatus for sealing a wound site. The apparatus comprising a sheet comprising a plurality of fixation strips and perforated portions. Each of the perforated portions can be positioned between adjacent fixation strips. Each of the perforated portions can comprise two perforated sections and a continuous cut section positioned between the two perforated sections. The two perforated sections can comprise a plurality of perforations separated by bridging portions that extend between adjacent fixation strips. Each of the plurality of fixation strips can comprise: an adhering layer, a first outer protective liner, a second outer protective liner, and a central protective liner. Each of the protective liners can be removably adhered to the adhering layer. The continuous cut section can extend along at least an entire length of the central protective liner.

In some embodiments, each of the plurality of fixation strips can be configured for placement over a portion of a wound dressing. The portion of the wound dressing can comprise an edge of the wound dressing.

In some embodiments, the adhering layer of each of the plurality of fixation strips can comprise an adhesive surface. In some embodiments, the adhesive surface can comprise a silicone adhesive configured to adhere to the wound site or to skin surrounding the wound site. In some embodiments, the adhesive surface can comprise an acrylic adhesive configured to adhere to a wound dressing.

In some embodiments, the first outer protective liner of each of the plurality of fixation strips can comprise a first release handle and the second outer protective liner comprises a second release handle. In some embodiments, the central protective liner of each of the plurality of fixation strips can be positioned over the first and second release handles.

In some embodiments, each of the plurality of fixation strips can comprise a wound sealing layer comprising a first adhesive. In some embodiments, the adhering layer can overly and extend outward beyond the wound sealing layer. In some embodiments, the adhering layer can comprise a second adhesive. In some embodiments, the first adhesive can comprises a silicone adhesive and the second adhesive can comprise an acrylic adhesive.

In some embodiments, each of the plurality of fixation strips can comprise a carrier layer removably adhered to a non-adhesive surface of the adhering layer. In some embodiments, the plurality of the fixation strips can be removably attached. In some embodiments, a length of each of the perforated portions can extend a length of each of the plurality of fixation strips.

### BRIEF DESCRIPTION OF THE DRAWINGS

FIG. 1 illustrates a reduced pressure wound therapy system according to some embodiments.
FIG. 2 illustrates a pump assembly and canister according to some embodiments.
FIG. 3 illustrates an electrical component schematic of a pump assembly according to some embodiments.
FIG. 4A illustrates an embodiment of a negative pressure wound treatment system comprising a pump, and illustrating a flexible suction adapter being applied to a wound.
FIG. 4B illustrates the embodiment of FIG. 4A, with the flexible suction adapter having been placed over a wound.
FIG. 5A illustrates an isometric view of a flexible suction adapter that may be used in a negative pressure wound treatment system.
FIG. 5B illustrates an exploded view of the flexible suction adapter of FIG. 5A.
FIG. 5C illustrates a close-up view of the proximal end of the flexible suction adapter of FIG. 5B.
FIG. 5D illustrates a close-up cutaway view of the proximal end of the flexible suction adapter of FIG. 2A.
FIG. 5E illustrates a top view of the flexible suction adapter of FIG. 5A.
FIG. 5F illustrates a side view of the flexible suction adapter of FIG. 5A.
FIG. 5G illustrates a bottom view of the flexible suction adapter of FIG. 5A.
FIG. 6 illustrates an exploded view of an alternative flexible suction adapter.
FIG. 7A illustrates an embodiment of a negative pressure wound treatment system employing a pump, a flexible fluidic connector and a wound dressing capable of absorbing and storing wound exudate.
FIG. 7B illustrates an embodiment of a negative pressure wound treatment system employing a flexible fluidic connector and a wound dressing capable of absorbing and storing wound exudate.
FIG. 7C illustrates a cross section of an embodiment of a fluidic connector connected to a wound dressing.
FIG. 8A illustrates an embodiment of a wound dressing incorporating a source of negative pressure and/or other electronic components within the wound dressing.
FIG. 8B illustrates an embodiment of layers of a wound dressing with the pump and electronic components offset from the absorbent area of the dressing.
FIG. 9A illustrates a cross-sectional side view along a length of an embodiment of a fixation strip.
FIG. 9B illustrates a cross-sectional side view along a width of an embodiment of the fixation strip shown in FIG. 9A.
FIG. 10A illustrates a top view of a dressing before applying a fixation strip.
FIG. 10B illustrates a top view of the dressing shown in FIG. 10A with a fixation strip.
FIGS. 11A-11F illustrate cross-sectional side views of an embodiment of a fixation strip as it is being applied to a dressing.
FIG. 12A illustrates a top view of an embodiment of a sheet of a plurality of fixation strips.
FIG. 12B illustrates a cross-sectional side view of the fixation strip shown in FIG. 12A.
FIG. 12C illustrates a close up view of a perforation pattern of the perforated sections of the sheet shown in FIG. 12A.
FIGS. 13A and 13B illustrate an exploded view and a perspective view of an embodiment of a drape according to certain aspects of the present disclosure.

### DETAILED DESCRIPTION

Embodiments disclosed herein relate to apparatuses, systems, devices and methods of treating a wound with reduced pressure. As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g., -40 mmHg is less than -60 mmHg). Negative pressure that is "more" or "greater" than -X mmHg corresponds to pressure that is further from atmospheric pressure (e.g., -80 mmHg is more than -60 mmHg). In some embodiments, local ambient atmospheric pressure is used as a reference point, and such local atmospheric pressure may not necessarily be, for example, 760 mmHg.

Embodiments of the present disclosure are generally applicable to use in topical negative pressure (TNP) or reduced pressure therapy systems. Briefly, negative pressure wound therapy assists in the closure and healing of many forms of "hard to heal" wounds by reducing tissue oedema, encouraging blood flow and granular tissue formation, or removing excess exudate and can reduce bacterial load (and thus infection risk). In addition, the therapy allows for less disturbance of a wound leading to more rapid healing. TNP therapy systems can also assist in the healing of surgically closed wounds by removing fluid. In some embodiments, TNP therapy helps to stabilize the tissue in the apposed position of closure. A further beneficial use of TNP therapy can be found in grafts and flaps where removal of excess fluid is important and close proximity of the graft to tissue is required in order to ensure tissue viability.

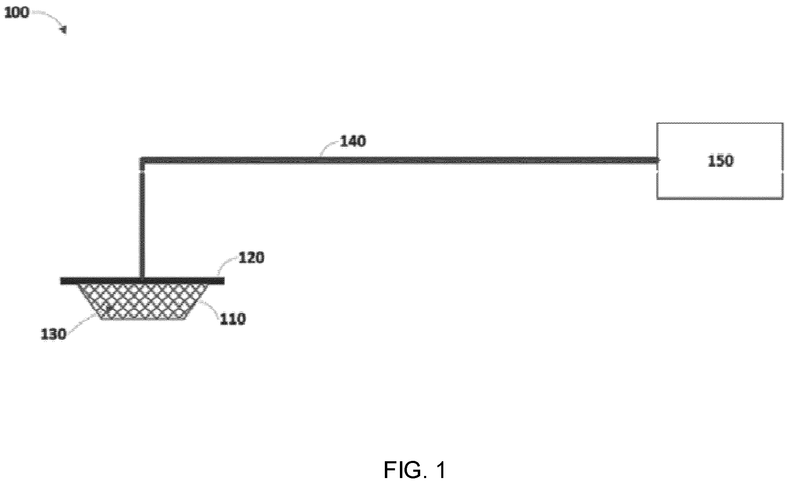

Embodiments described herein relate to materials, apparatuses, methods, and systems that incorporate, or comprise, or utilize a wound contact layer ("WCL") for positioning in contact with a wound. A WCL may be utilized as a stand-alone component for separately positioning at a wound site, or may be incorporated into any number of multi-layer wound dressings and wound treatment apparatuses, such as described herein below with respect to Figs. 1 through 9. Embodiments of the present disclosure are generally applicable to use under ambient conditions, in negative pressure or reduced pressure therapy systems, or in compression therapy systems.

Some of the preferred embodiments described herein incorporate, or comprise, or utilize multi-care wound contact layers. Such a multi-care WCL possesses two or more of the following functional features: antimicrobial activities, easiness to apply or/and remove as one piece, easiness to cut with scissors, conformability to the three-dimensional contour of a wound surface, durability to wear, compatibility with negative pressure wound therapy or/and compression wound therapy, exudate management, capability of facilitating autolytic debridement of wound, capability of promoting wound healing, and self-indication of compositional or functional changes.

Certain embodiments described herein provide a wound treatment system. Such a wound treatment system may comprise a stand-alone layer of multi-care WCL, configured to be sized for positioning over a wound. The wound treatment system may further comprise a secondary wound dressing configured to be separately positioned over the multi-care WCL. The multi-care WCL may have an adhesive adhered to the lower surface; and the adhesive can be configured that the multi-care WCL will be placed in proximity to the wound. The secondary wound dressing, if used, may adhere to skin surrounding the wound and may have the same size or may be larger than the multi-care WCL, so that the multi-care WCL will touch or be placed in proximity to the wound. The secondary wound dressing can be alternatively or additionally configured to form a seal to skin surrounding the wound so that the multi-care WCL will touch or be placed in proximity to the wound. The wound treatment system may further comprise a source of negative pressure configured to supply negative pressure through the secondary wound dressing and through the multi-care wound contact layer to the wound.

Certain other embodiments described herein provide a multi-layered wound dressing, such as described herein the specification with respect to Figs. 1 through 9. Such a multi-layered wound dressing may incorporate a multi-care WCL as a component layer thereof or, alternatively, may comprise a composite or laminate including the multi-care WCL as part of one of the component layers thereof. The multi-layered wound dressing may comprise: a multi-care wound contact layer as described above or described elsewhere herein; a transmission layer and/or absorbent layer over the multi-care wound contact layer; and a cover layer over the transmission layer and/or absorbent layer. The wound dressing may further comprise an adhesive layer on the lower surface of the multi-care wound contact layer. The wound dressing may further comprise a negative pressure port positioned on or above the cover layer. The multi-care wound contact layer may have a perimeter shape that is substantially the same as a perimeter shape of the cover layer. Alternatively, the multi-care wound contact layer may have a perimeter shape that is smaller than a perimeter shape of the cover layer.

Some embodiments described herein the specification provide a method to treat a wound or locus. Such a method may include placing a multi-care WCL, either separately or by placing a multi-layered wound dressing having a multi-care WCL, over the wound. The method may comprise adhering the separate multi-care WCL and/or the multi-layer wound dressing having a multi-care WCL to healthy skin around the wound. The method may further comprise one or more of the following steps: A further wound dressing can be placed over the separate multi-care WCL or multi-layered wound dressing having the multi-care WCL that is placed over the wound. Wound exudate, or any moist or aqueous medium other than wound exudate, may be provided to reach and/or touch the multi-care WCL. Wound exudate, or any moist or aqueous medium other than wound exudate may be diffused or wicked into the wound dressing incorporating the multi-care WCL or into a wound dressing provided over the multi-care WCL. Negative pressure may be applied to the separate multi-care WCL or multi-layered wound dressing having the multi-care WCL, such that wound exudate is suctioned into the multi-care WCL directly, or into the wound dressing incorporating the multi-care WCL, or into a wound dressing provided over the multi-care WCL.

### Negative Pressure System

Fig. 1 illustrates an embodiment of a negative or reduced pressure wound treatment (or TNP) system 100 including a wound filler 130 placed inside a wound cavity 110, the wound cavity sealed by a wound cover 120. The wound filler 130 in combination with the wound cover 120 can be referred to as wound dressing. A flow path 140, such as a single or multi lumen tube or conduit, is connected to the wound cover 120 with a negative pressure wound therapy device, for example pump assembly 150, configured to supply reduced pressure. The wound cover 120 can be in fluidic communication with the wound cavity 110. In any of the system embodiments disclosed herein, as in the embodiment illustrated in Fig. 1, the pump assembly can be a canisterless pump assembly (meaning that exudate is collected in the wound dressing or is transferred via tube 140 for collection to another location). However, any of the pump assembly embodiments disclosed herein can be configured to include or support a canister. Additionally, in any of the system embodiments disclosed herein, any of the pump assembly embodiments can be mounted to or supported by the dressing, or adjacent to the dressing. The wound filler 130 can be any suitable type, such as hydrophilic or hydrophobic foam, gauze, inflatable bag, and so on. The wound filler 130 can be conformable to the wound cavity 110 such that it substantially fills the cavity. The wound cover 120 can provide a substantially fluid impermeable seal over the wound cavity 110. The wound cover 120 can have a top side and a bottom side, and the bottom side adhesively (or in any other suitable manner) seals with wound cavity 110. The conduit 140 or lumen or any other conduit or lumen disclosed herein can be formed from polyurethane, PVC, nylon, polyethylene, silicone, or any other suitable material.

Some embodiments of the wound cover 120 can have a port (not shown) configured to receive an end of the conduit 140. In other embodiments, the conduit 140 can otherwise pass through or under the wound cover 120 to supply reduced pressure to the wound cavity 110 so as to maintain a desired level of reduced pressure in the wound cavity. The conduit 140 can be any suitable article configured to provide at least a substantially sealed fluid flow pathway between the pump assembly 150 and the wound cover 120, so as to supply the reduced pressure provided by the pump assembly 150 to wound cavity 110. The wound cover 120 and the wound filler 130 can be provided as a single article or an integrated single unit. In some embodiments, no wound filler is provided and the wound cover by itself may be considered the wound dressing. The wound dressing may then be connected, via the conduit 140, to a source of negative pressure, such as the pump assembly 150. The pump assembly 150 can be miniaturized and portable, although larger conventional pumps such can also be used.

The wound cover 120 can be located over a wound site to be treated. The wound cover 120 can form a substantially sealed cavity or enclosure over the wound site. In some embodiments, the wound cover 120 can be configured to have a film having a high water vapour permeability to enable the evaporation of surplus fluid, and can have a superabsorbing material contained therein to safely absorb wound exudate. It will be appreciated that throughout this specification reference is made to a wound. In this sense it is to be understood that the term wound is to be broadly construed and encompasses open and closed wounds in which skin is torn, cut or punctured or where trauma causes a contusion, or any other surficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, acute wounds, chronic wounds, surgical incisions and other incisions, subacute and dehisced wounds, traumatic wounds, flaps and skin grafts, lacerations, abrasions, contusions, burns, diabetic ulcers, pressure ulcers, stoma, surgical wounds, trauma and venous ulcers or the like. The components of the TNP system described herein can be particularly suited for incisional wounds that exude a small amount of wound exudate.

Some embodiments of the system are designed to operate without the use of an exudate canister. Some embodiments can be configured to support an exudate canister. In some embodiments, configuring the pump assembly 150 and tubing 140 so that the tubing 140 can be quickly and easily removed from the pump assembly 150 can facilitate or improve the process of dressing or pump changes, if necessary. Any of the pump embodiments disclosed herein can be configured to have any suitable connection between the tubing and the pump.

In some embodiments, the pump assembly 150 can be configured to deliver negative pressure of approximately -80 mmHg, or between about -20 mmHg and -200 mmHg. Note that these pressures are relative to normal ambient atmospheric pressure thus, -200 mmHg would be about 560 mmHg in practical terms. The pressure range can be between about -40 mmHg and -150 mmHg. Alternatively a pressure range of up to -75 mmHg, up to -80 mmHg or over -80 mmHg can be used. Also a pressure range of below -75 mmHg can be used. Alternatively a pressure range of over approximately -100 mmHg, or even 150 mmHg, can be supplied by the pump assembly 150. In some embodiments, the pump assembly 150 is configured to provide continuous or intermittent negative pressure therapy. Continuous therapy can be delivered at above -25 mmHg, -25 mmHg, -40 mmHg, -50 mmHg, -60 mmHg, -70 mmHg, -80 mmHg, -90 mmHg, -100 mmHg, -120 mmHg, -140 mmHg, -160 mmHg, -180 mmHg, -200 mmHg, or below -200 mmHg. Intermittent therapy can be delivered between low and high negative pressure setpoints. Low setpoint can be set at above 0 mmHg, 0 mmHg, -25 mmHg, -40 mmHg, -50 mmHg, -60 mmHg, -70 mmHg, -80 mmHg, -90 mmHg, -100 mmHg, -120 mmHg, -140 mmHg, -160 mmHg, -180 mmHg, or below -180 mmHg. High setpoint can be set at above -25 mmHg, -40 mmHg, -50 mmHg, -60 mmHg, -70 mmHg, -80 mmHg, -90 mmHg, -100 mmHg, -120 mmHg, -140 mmHg, -160 mmHg, -180 mmHg, -200 mmHg, or below -200 mmHg. During intermittent therapy, negative pressure at low setpoint can be delivered for a first-time duration, and upon expiration of the first-time duration, negative pressure at high setpoint can be delivered for a second-time duration. Upon expiration of the second-time duration, negative pressure at low setpoint can be delivered. The first and second time durations can be same or different values. The first and second durations can be selected from the following range: less than 2 minutes, 2 minutes, 3 minutes, 4 minutes, 6 minutes, 8 minutes, 10 minutes, or greater than 10 minutes. In some embodiments, switching between low and high setpoints and vice versa can be performed according to a step waveform, square waveform, sinusoidal waveform, and the like.

In some embodiments, the TNP system 100 can include multiple wound dressings connected to the pump assembly 150. The performance and wound healing capabilities (such as, fluid management) of the TNP system with multiple wound dressings with the pump assembly 150 can be equivalent to or exceed that of a standard single wound dressing with single pump set-up.

In operation, the wound filler 130 is inserted into the wound cavity 110 and wound cover 120 is placed so as to seal the wound cavity 110. The pump assembly 150 provides a source of a negative pressure to the wound cover 120, which is transmitted to the wound cavity 110 via the wound filler 130. Fluid (e.g., wound exudate) is drawn through the conduit 140, and can be stored in a canister. In some embodiments, fluid is absorbed by the wound filler 130 or one or more absorbent layers (not shown).

Wound dressings that may be utilized with the pump assembly and other embodiments of the present application include Renasys-F, Renasys-G, Renasys AB, and Pico Dressings available from Smith & Nephew. Any of the dressings described herein can be used with Smith and Nephew's Renasys Soft Port connector or interface between the dressing and the pump assembly. For example, the Renasys Soft Port connector can be positioned in the flow path 140 and serve as a port for the wound dressing. In other embodiments, other suitable wound dressings can be utilized.

### Pump Assembly and Canister

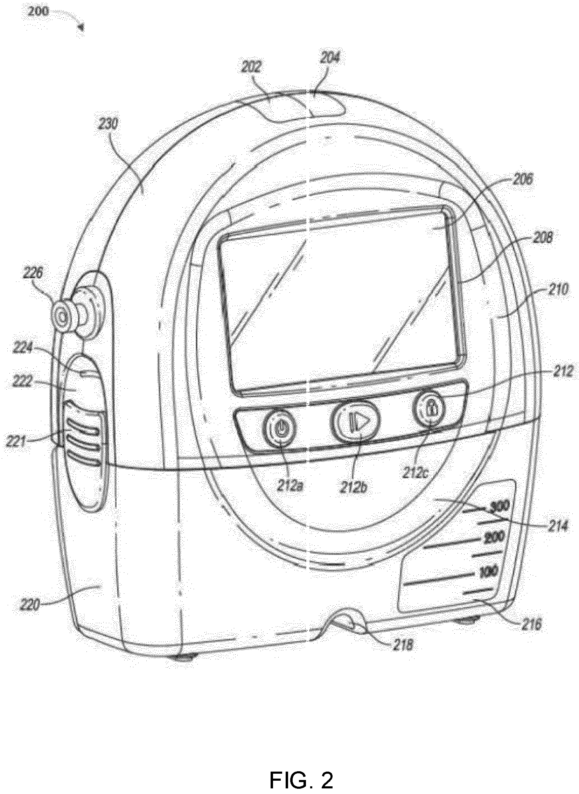

Fig. 2 illustrates a front view 200 of a pump assembly 230 and canister 220 according to some embodiments. As is illustrated, the pump assembly 230 and the canister are connected, thereby forming a TNP device or system. The pump assembly 230 includes one or more indicators, such as visual indicator 202 configured to indicate alarms and visual indicator 204 configured to indicate status of the TNP system. The indicators 202 and 204 can be configured to alert a user, such as patient or medical care provider, to a variety of operating or failure conditions of the system, including alerting the user to normal or proper operating conditions, pump failure, power supplied to the pump or power failure, detection of a leak within the wound cover or flow pathway, suction blockage, no flow condition, canister full condition, or any other similar or suitable conditions or combinations thereof. The pump assembly 230 can include additional indicators. The pump assembly can use a single indicator or multiple indicators. Any suitable indicator can be used such as visual, audio, tactile indicator, and so on. The indicator 202 can be configured to signal alarm conditions, such as canister full, power low, conduit 140 disconnected, seal broken in the wound seal 120, and so on. The indicator 202 can be configured to display red flashing light to draw user's attention. The indicator 204 can be configured to signal status of the TNP system, such as therapy delivery is ok, leak detected, and so on. The indicator 204 can be configured to display one or more different colors of light, such as green, yellow, etc. For example, green light can be emitted when the TNP system is operating properly and yellow light can be emitted to indicate a warning.

The pump assembly 230 may include a display or screen 206 mounted in a recess 208 formed in a case of the pump assembly. The display 206 can be a touch screen display. The display 206 can support playback of audiovisual (AV) content, such as instructional videos. As explained herein, the display 206 can be configured to render a number of screens or graphical user interfaces (GUIs) for configuring, controlling, and monitoring the operation of the TNP system. The pump assembly 230 includes a gripping portion 210 formed in the case of the pump assembly. The gripping portion 210 can be configured to assist the user to hold the pump assembly 230, such as during removal of the canister 220. The canister 220 can be replaced with another canister, such as when the canister 220 has been filled with fluid.

The pump assembly 230 may include one or more keys or buttons 212 configured to allow the user to operate and monitor the operation of the TNP system. As is illustrated, there buttons 212a, 212b, and 212c are included. Button 212a can be configured as a power button to turn on/off the pump assembly 230. Button 212b can be configured as a play/pause button for the delivery of negative pressure therapy. For example, pressing the button 212b can cause therapy to start, and pressing the button 212b afterward can cause therapy to pause or end. Button 212c can be configured to lock the display 206 or the buttons 212. For instance, button 212c can be pressed so that the user does not unintentionally alter the delivery of the therapy. Button 212c can be depressed to unlock the controls. In other embodiments, additional buttons can be used or one or more of the illustrated buttons 212a, 212b, or 212c can be omitted. Multiple key presses or sequences of key presses can be used to operate the pump assembly 230.

The pump assembly 230 may include one or more latch recesses 222 formed in the cover. In the illustrated embodiment, two latch recesses 222 can be formed on the sides of the pump assembly 230. The latch recesses 222 can be configured to allow attachment and detachment of the canister 220 using one or more canister latches 221. The pump assembly 230 includes an air outlet 224 for allowing air removed from the wound cavity 110 to escape. Air entering the pump assembly can be passed through one or more suitable filters, such as antibacterial filters. This can maintain reusability of the pump assembly. The pump assembly 230 includes one or more strap mounts 226 for connecting a carry strap to the pump assembly 230 or for attaching a cradle. In the illustrated embodiment, two strap mounts 226 can be formed on the sides of the pump assembly 230. In some embodiments, various features are omitted or various additional features are added to the pump assembly 230.

The canister 220 may be configured to hold fluid (e.g., exudate) removed from the wound cavity 110. The canister 220 includes one or more latches 221 for attaching the canister to the pump assembly 230. In the illustrated embodiment, the canister 220 includes two latches 221 on the sides of the canister. The exterior of the canister 220 can formed from frosted plastic so that the canister is substantially opaque and the contents of the canister and substantially hidden from plain view. The canister 220 includes a gripping portion 214 formed in a case of the canister. The gripping portion 214 can be configured to allow the user to hold the pump assembly 220, such as during removal of the canister from the apparatus 230. The canister 220 includes a substantially transparent window 216, which can also include graduations of volume. For example, the illustrated 300 mL canister 220 includes graduations of 50 mL, 100 mL, 150 mL, 200 mL, 250 mL, and 300 mL. Other embodiments of the canister can hold different volume of fluid and can include different graduation scale. For example, the canister can be an 800 mL canister. The canister 220 includes a tubing channel 218 for connecting to the conduit 140. In some embodiments, one or more of these features, such as the gripping portion 214, are omitted or various additional features are added to the canister 220. Any of the disclosed canisters may include or may omit a solidifier.

### Electronics and Software

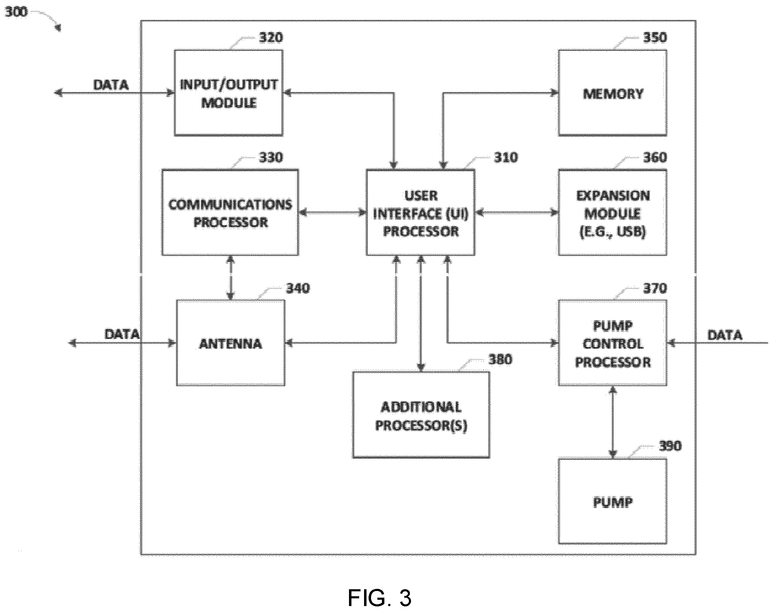

Fig. 3 illustrates an electrical component schematic 300 of a pump assembly, such as the pump assembly 230, according to some embodiments. Electrical components can operate to accept user input, provide output to the user, operate the pump assembly and the TNP system, provide network connectivity, and so on. Electrical components can be mounted on one or more printed circuit boards (PCBs). As is illustrated, the pump assembly can include multiple processors. It may be advantageous to utilize multiple processors in order to allocate or assign various tasks to different processors. A first processor can be responsible for user activity and a second processor can be responsible for controlling the pump. This way, the activity of controlling the pump, which may necessitate a higher level of responsiveness (corresponding to higher risk level), can be offloaded to a dedicated processor and, thereby, will not be interrupted by user interface tasks, which may take longer to complete because of interactions with the user.

The pump assembly can include a user interface processor or controller 310 configured to operate one or more components for accepting user input and providing output to the user, such as the display 206, buttons 212, etc. Input to the pump assembly and output from the pump assembly can controlled by an input/output (I/O) module 320. For example, the I/O module can receive data from one or more ports, such as serial, parallel, hybrid ports, and the like. The processor 310 also receives data from and provides data to one or more expansion modules 360, such as one or more USB ports, SD ports, Compact Disc (CD) drives, DVD drives, FireWire ports, Thunderbolt ports, PCI Express ports, and the like. The processor 310, along with other controllers or processors, stores data in one or more memory modules 350, which can be internal or external to the processor 310. Any suitable type of memory can be used, including volatile or non-volatile memory, such as RAM, ROM, magnetic memory, solid-state memory, Magnetoresistive random-access memory (MRAM), and the like.

In some embodiments, the processor 310 can be a general-purpose controller, such as a low-power processor. In other embodiments, the processor 310 can be an application specific processor. The processor 310 can be configured as a "central" processor in the electronic architecture of the pump assembly, and the processor 310 can coordinate the activity of other processors, such as a pump control processor 370, communications processor 330, and one or more additional processors 380 (e.g., processor for controlling the display 206, processor for controlling the buttons 212, etc.). The processor 310 can run a suitable operating system, such as a Linux, Windows CE, VxWorks, etc.

The pump control processor 370 can be configured to control the operation of a negative pressure source or pump 390. The pump 390 can be a suitable pump, such as a diaphragm pump, peristaltic pump, rotary pump, rotary vane pump, scroll pump, screw pump, liquid ring pump, pump (for example, diaphragm pump) operated by a piezoelectric transducer, voice coil pump, and the like. The pump control processor 370 can measure pressure in a fluid flow path, using data received from one or more pressure sensors, calculate the rate of fluid flow, and control the pump. The pump control processor 370 can control an actuator, such as a pump motor, so that a desired level of negative pressure is achieved in the wound cavity 110. The desired level of negative pressure can be pressure set or selected by the user. In various embodiments, the pump control processor 370 controls the pump actuator (e.g., pump motor) using pulse-width modulation (PWM). A control signal for driving the pump actuator can be a 0-100% duty cycle PWM signal. The pump control processor 370 can perform flow rate calculations and detect various conditions in a flow path. The pump control processor 370 can communicate information to the processor 310. The pump control processor 370 can include internal memory or can utilize memory 350. The pump control processor 370 can be a low-power processor.

A communications processor 330 can be configured to provide wired or wireless connectivity. The communications processor 330 can utilize one or more antennas 340 for sending and receiving data. The communications processor 330 can provide one or more of the following types of connections: Global Positioning System (GPS) technology, cellular connectivity (e.g., 2G, 3G, LTE, 4G), Wi-Fi connectivity, Internet connectivity, and the like. Connectivity can be used for various activities, such as pump assembly location tracking, asset tracking, compliance monitoring, remote selection, uploading of logs, alarms, and other operational data, and adjustment of therapy settings, upgrading of software or firmware, and the like. The communications processor 330 can provide dual GPS/cellular functionality. Cellular functionality can, for example, be 3G functionality. In such cases, if the GPS module is not able to establish satellite connection due to various factors including atmospheric conditions, building or terrain interference, satellite geometry, and so on, the device location can be determined using the 3G network connection, such as by using cell identification, triangulation, forward link timing, and the like. The pump assembly can include a SIM card, and SIM-based positional information can be obtained.

The communications processor 330 can communicate information to the processor 310. The communications processor 330 can include internal memory or can utilize memory 350. The communications processor 330 can be a low-power processor.

In some embodiments, the pump assembly can track and store various data, such as one or more of positioning data, therapy parameters, logs, device data, and so on. The pump assembly can track and log therapy and other operational data. Data can be stored, for example, in the memory 350.

In some embodiments, using the connectivity provided by the communications processor 330, the device can upload any of the data stored, maintained, or tracked by the pump assembly. For example, the following information can be uploaded to a remote computer or server: activity log(s), which includes therapy delivery information, such as therapy duration, alarm log(s), which includes alarm type and time of occurrence; error log, which includes internal error information, transmission errors, and the like; therapy duration information, which can be computed hourly, daily, and the like; total therapy time, which includes therapy duration from first applying a particular therapy program or programs; lifetime therapy information; device information, such as the serial number, software version, battery level, etc.; device location information; patient information; and so on. The device can also download various operational data, such as therapy selection and parameters, firmware and software patches and upgrades, and the like. The pump assembly can provide Internet browsing functionality using one or more browser programs, mail programs, application software (e.g., apps), etc.

In some embodiments, the communications processor 330 can use the antenna 340 to communicate a location of the pump assembly, such as a location of a housing of the pump assembly, to other devices in the proximity (for example, within 10, 20, or 50 meters and the like) of the pump assembly. The communications processor 330 can perform one-way or two-way communication with the other devices depending on the implementation. The communications transmitted by the communications processor 330 can include identifying information to uniquely identify the pump assembly relative to one or more other pump assemblies also in the proximity of the pump assembly. For example, identifying information can include a serial number or a value derived from the serial number. The signal strength of the transmitted communications by the communications processor 330 can be controlled (for example, maintained at a constant or substantially constant level) to enable another device to determine a distance to the pump assembly, such as a distance between the device and the pump assembly.

In some embodiments, the communications processor 330 can communicate with other devices in the proximity of the pump assembly so that the communications processor 330 can itself determine a distance from the pump assembly to the other devices. The communications processor 330, in such embodiments, can track and store the distance from the pump assembly to the other devices or indications of change in the distance over time, and the communications processor 330 can later provide this information to the other devices. For instance, the communications processor 330 can determine a duration of time during which the pump assembly has been removed from a coverage area of a device and subsequently report this time to the device upon being returned to the coverage area.

### Flexible Suction Adapter

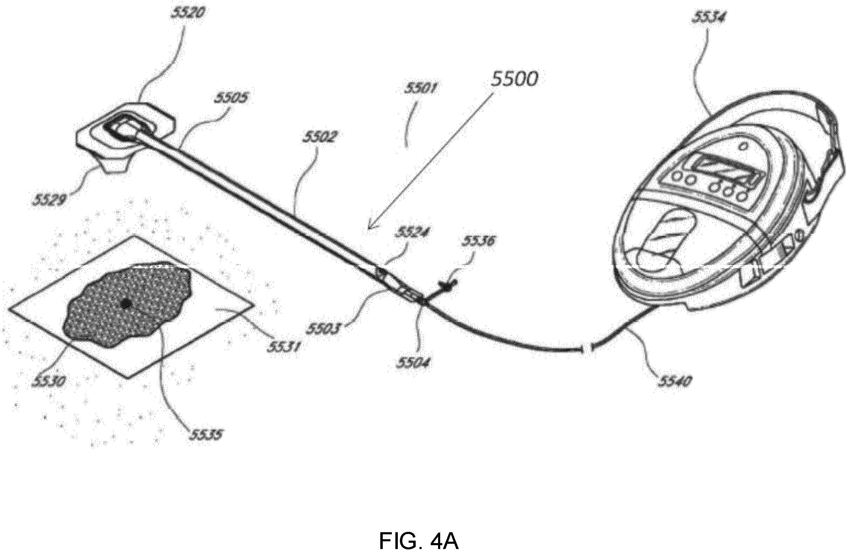

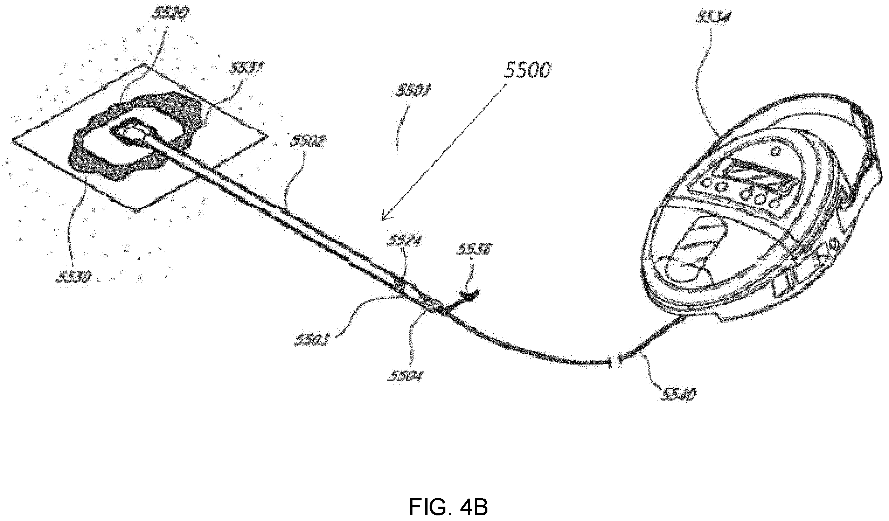

Figs. 4A-6 illustrate embodiments of a negative pressure wound treatment system 5501 similar to the embodiment illustrated in Fig. 1. Here, the system 5501 may comprise a flexible suction adapter 5500 having a bridge portion 5502 with a proximal end 5503 and a distal end 5505, and an applicator 5520 at the distal end 5505 of the bridge portion 5502 forming the flexible suction adapter 5500. A connector 5504 is preferably disposed at the proximal end 5503 of the bridge portion 5502, so as to connect to at least one of the channels 5512 and/or 5516, as shown in Fig. 4B. A cap 5536 may be provided with the system 5501 (and can in some cases, as illustrated, be attached to the connector 5504). The cap 5536 can be useful in preventing fluids from leaking out of the proximal end 5503. The system 5501 may include a source of negative pressure such as a pump or negative pressure unit 5534 capable of supplying negative pressure. The pump also preferably comprises a canister or other container for the storage of wound exudates and other fluids that may be removed from the wound. In some embodiments, this pump 5534 may be the pump 200 described in relation to Fig. 2. In some embodiments, this pump 5534 can be a RENASYS GO pump, as sold by Smith & Nephew. The pump 5534 may be connected to the connector 5504 via a tube 5540. In use, the applicator 5520 is placed over an aperture 5535 formed in a drape 5531 that is placed over a suitably-prepared wound 5530, which may in some cases be filled with a wound packing material such as foam or gauze. Subsequently, with the pump 5534 connected via the tube 5540 to the connector 5504, the pump is activated, thereby supplying negative pressure to the wound. Application of negative pressure may be applied until a desired level of healing of the wound 5530 is achieved.

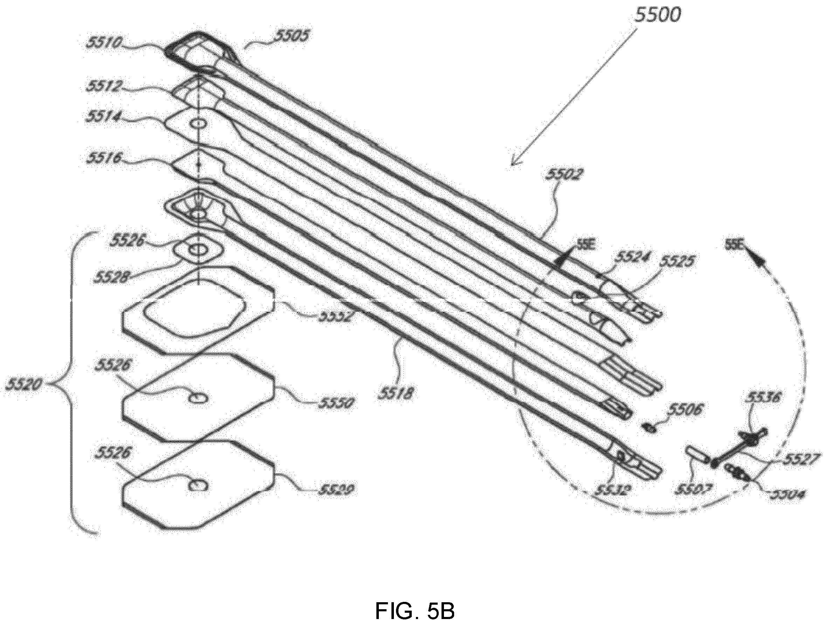

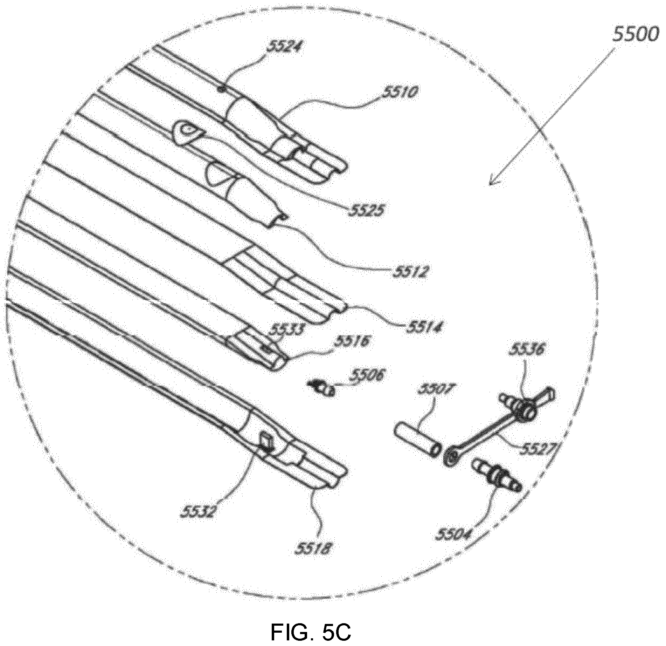

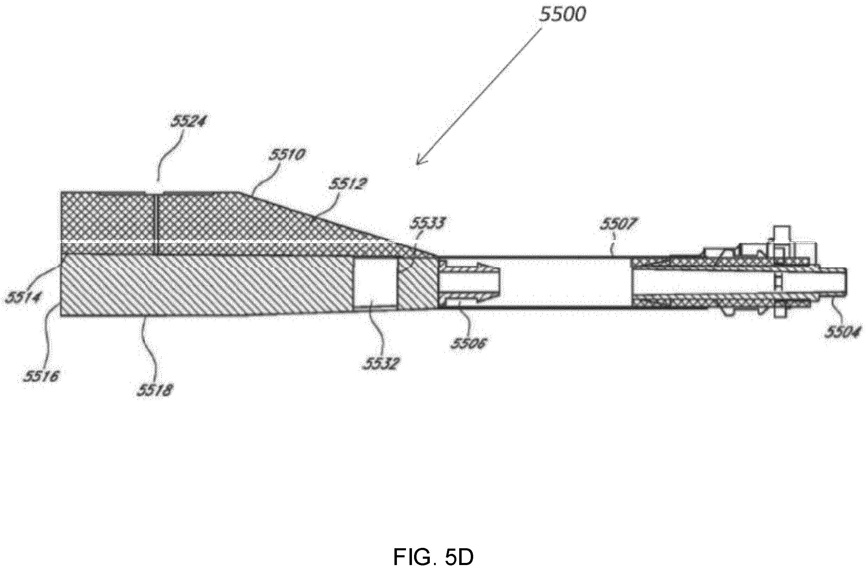

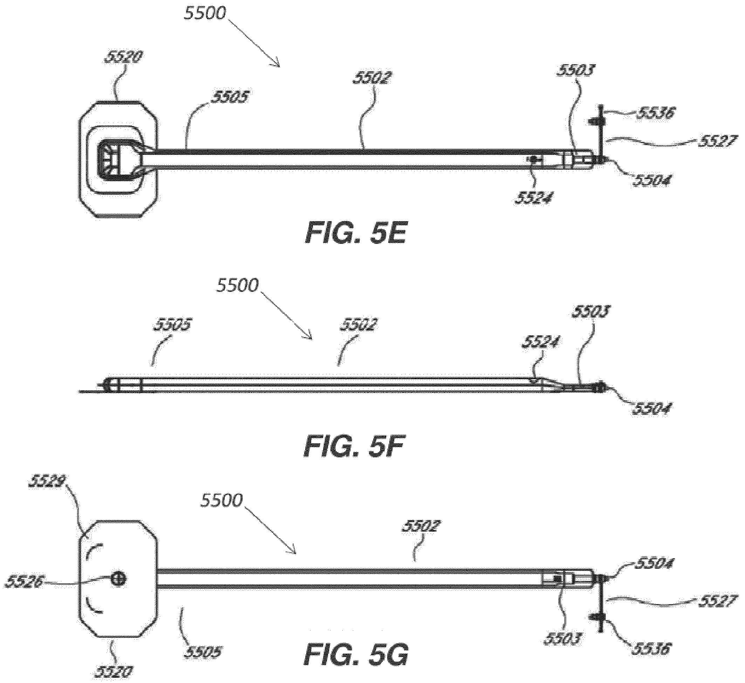

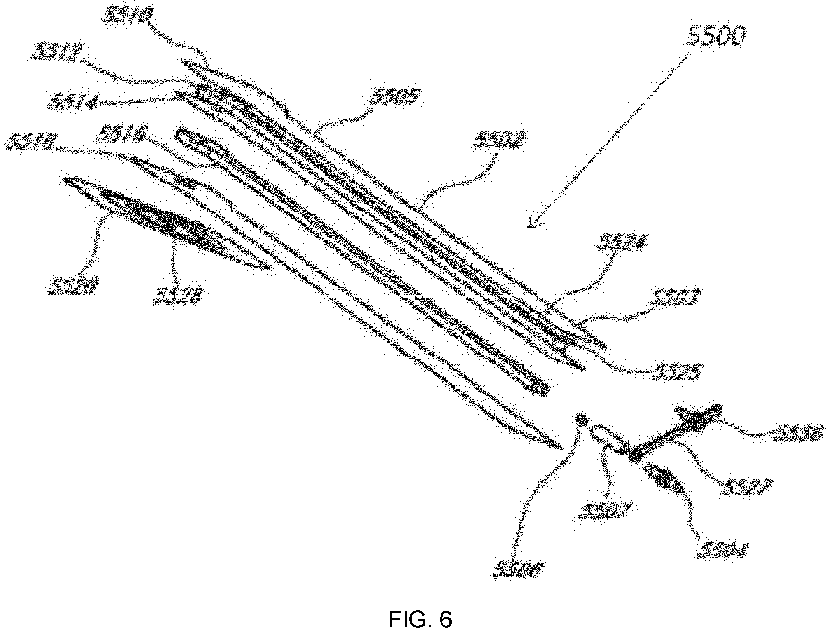

In some embodiments, the bridge portion 5502 may comprise an upper channel layer 5512 positioned between an upper layer 5510 and an intermediate layer 5514, with a lower channel layer 5516 positioned between the intermediate layer 5514 and a bottom layer 5518. Preferably, the layers 5510, 5514, and 5518 have elongate portions extending between proximal and distal ends and may be comprised of a material that is fluid-impermeable, for example polymers such as polyurethane. It will of course be appreciated that the layers 5510, 5514, and 5518 may each be constructed from different materials, including semi-permeable materials. In some embodiments, one or more of the layers 5510, 5514, and 5518 may be at least partially transparent. As illustrated in FIG. 5B, the upper and lower layers 5510 and 5518 may be curved, rounded or outwardly convex over a majority of their lengths. During assembly, for example, the layers 5510, 5514, and 5518 may be pinched together to weld or adhere the layers together. In doing so, the proximal ends of the channels 5512 and 5516 may be sandwiched between these layers, thus partially compressing the proximal ends of the channels 5512, 5516 and stretching the layers 5510, 5514, 5518 over these aforementioned proximal ends. Of course, the proximal ends of the materials used in the bridge portion 5502 may not necessarily be rounded or curved; as shown in Fig. 6, they can remain substantially squared off and straight.

The upper and lower channel layers 5512 and 5516 are preferably elongate layers extending from the proximal end 5503 to the distal end 5505 and may each preferably comprise a porous material, including for example open-celled foams such as polyethylene or polyurethane. In some embodiments, one or more of the upper and lower channel layers 5512 and 5516 may be comprised of a fabric, for example a knitted or woven spacer fabric (such as a knitted polyester 3D fabric, Baltex 7970.RTM., or Gehring 879.RTM.) or a nonwoven material. Suitable materials may also include terry-woven or loop-pile materials. The fibers may not necessarily be woven, and can include felted and flocked (including materials such as Flotex.RTM.) fibrous materials. The materials selected are preferably suited to channeling wound exudate away from the wound and for transmitting negative pressure and/or vented air to the wound site, and may also confer a degree of kinking or occlusion resistance to the channel layers 5512 and 5516 as described below. In one embodiment, the upper channel layer 5512 may comprise an open-celled foam such as polyurethane, and the lower channel layer may comprise a fabric as described herein. In another embodiment, the upper channel layer is optional, and the system may instead be provided with an open upper channel. In the embodiment illustrated in Fig. 5B, the upper channel layer 5512 may have a curved, rounded or upwardly convex upper surface and a substantially flat lower surface, and the lower channel layer 5516 may have a curved, rounded or downwardly convex lower surface and a substantially flat upper surface.

In some embodiments, the fabric may have a three-dimensional (3D) structure, where one or more types of fibers form a structure where the fibers extend in all three dimensions. Such a fabric may in some cases aid in wicking, transporting fluid, and/or transmitting negative pressure. To prevent the channels 5512 and/or 5516 from being displaced or twisted while encased in the system 5501--which may impair performance of the respective channels under negative pressure--it may in some embodiments be preferable to adhere or otherwise secure the channels 5512 and/or 5516 to one or more of the layers 5510, 5514, and 5518. In certain embodiments, these materials remain open and capable of communicating negative pressure to a wound area under the typical pressures used in negative pressure therapy, for example between 40 to 150 mmHg, although higher and lower values are possible. In some embodiments, the fabric may comprise several layers of material stacked or layered over each other, which may in some cases be useful in preventing the channel 5516 from collapsing under the application of negative pressure. In other embodiments, the fabric used in channel 5516 may be between 1.5 mm and 6 mm; more preferably, the fabric may be between 3 mm and 6 mm thick, and may be comprised of either one or several individual layers of fabric. In other embodiments, the channel 5512 may be between 1.2-3 mm thick, and preferably thicker than 1.5 mm. Additionally, and as described previously, the materials used in the system 5501 are preferably conformable and soft, which may help to avoid pressure ulcers and other complications which may result from a wound treatment system being pressed against the skin of a patient.

In some embodiments, the distal ends of the layers 5510, 5514, and 5518 and the channel layers 5512 and 5516 are enlarged at their distal ends (to be placed over a wound site), and may form a "teardrop" or other enlarged shape. The distal ends of at least the layers 5512, 5514, 5516, and 5518 may also be provided with at least one through aperture. This aperture may be useful not only for the drainage of wound exudate and for applying negative pressure to the wound, but also during manufacturing of the device, as these apertures may be used to align these respective layers appropriately.

With additional reference to Figs. 5B-C and 6, a channel connector 5506 may be provided at the proximal end 5503 of the bridge portion 5502, the channel connector 5506 preferably being configured so as to be embedded into the lower channel layer 5516 so as to create a secure fluidic connection. The channel connector 5506 may in some embodiments be inserted into a pre-made cavity formed into the channel 5516; as illustrated in Fig. 6, this cavity can be cut out or can be in the form of a rabbet joint. With one end of the channel connector 5506 being embedded into the lower channel layer 5516, the other end of the channel connector 5506 may be connected or in communication with, in one embodiment, a connector tube 5507, although in some embodiments the channel connector 5506 may be connected directly to the connector 5504, or else connected directly to a tube 5540 connected to a source of negative pressure. When using a connector tube 5507, the resulting assembly can permit a connector 5504 to be attached thereto. A cap 5536, which may be secured to the suction adapter for example via a cap leash 5527 secured with a ring disposed on the outer surface of the connector tube 5507. The cap 5536 may be used to cover the end of the suction adapter, for example at the connector 5504, so as to prevent exudate and other wound fluids from leaking out. The connector 5504 is preferably configured to connect with a tube 5540 connected to a source of negative pressure. The connector 5504 may for example comprise a lip or other such structure to aid in securing the connector 5504 to a tube 5540 and/or cap 5536, although it will be understood that other connector types are possible, including quick-disconnect couplings, luer locks, Christmas-tree, and other such connectors.

The upper layer 5510 may comprise additional material extending downward, preferably at least of the thickness of the bridge portion 5502; this material may then be used to bond or weld to the other layers so to form a fluid-tight seal. More specifically, during assembly, the upper layer 5510 may be attached, for example by melting, welding, or with adhesives, to the lower layer 5518 so as to form a fluid-tight seal (with the exception of the apertures at the distal and proximal ends). Preferably, the middle layer 5514 is attached to the top layer 5510 and the bottom layer 5518. In some embodiments, it may be preferable to attach or bond the connectors 5504 and/or 5506, as well as the tube 5507 to at least one of the layers 5510, 5514, 5518 so as to create a fluid-tight connection. To provide for a more secure connection, some embodiments may also be provided with a weld 5532 made onto the lower layer 5518. The lower channel 5516 may have a hole or aperture made through it, which may be used to weld it, via the weld 5532, to the lower layer 5518. This welding of the lower channel 5516 to the lower layer 5518 via the weld 5532 made through the hole 5533 may thus aid in preventing the various layers and channels from shifting or being displaced. Obviously, it will be understood that other securement means may be used, for example adhesives and the like, and that such arrangements may be also be used in the upper channel 5512.

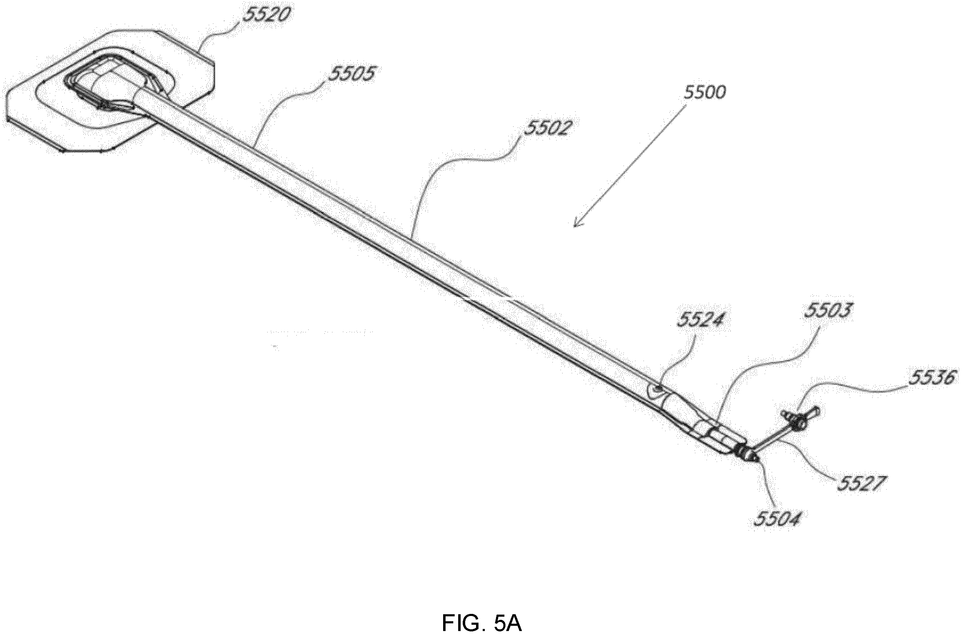

In certain embodiments, for example as illustrated in Figs. 5A-6, a controlled air leak 5524 may be disposed on the bridge portion 5502, for example at the proximal end thereof. This air leak 5524 may comprise an opening or channel extending through upper layer 5510, such that the air leak 5524 is in fluidic communication with the upper channel 5512. Upon the application of suction to the suction adapter 5500, air will enter through the air leak 5524 and move from the proximal end 5503 to the distal end 5505 along the upper channel 5512. The air will then be suctioned into the lower channel 5516 by passing through the apertures through the distal ends of the layers 5512, 5514, 5516 and 5518. The air leak 5524 preferably comprises a filter 5525. Preferably, the air leak 5524 is located at the proximal end of the bridge portion 5502 so as to minimize the likelihood of wound exudate or other fluids coming into contact and possibly occluding or interfering with the air leak 5524 or its filter 5525. In some embodiments, this filter 5525 is a microporous membrane capable of excluding microorganisms and bacteria, and which may be able to filter out particles larger than 45 µm. Preferably, the filter 5525 can exclude particles larger than 1.0 µm, and more preferably, particles larger than 0.2 µm. Advantageously, some embodiments may provide for a filter 5525 that is at least partially chemically-resistant, for example to water, common household liquids such as shampoos, and other surfactants. In some embodiments, reapplication of vacuum to the suction adapter 5500 and/or wiping of the exposed outer portion of the filter 5525 may be sufficient to clear any foreign substance occluding the filter 5525. The filter 5525 may be composed of a suitably-resistant polymer such as acrylic, polyethersulfone, or polytetrafluoroethylene, and may be oleophobic and/or hydrophobic. In some embodiments, the filter 5525 may also comprise a supporting backing layer, for example a nonwoven polyester support. Preferably, the air leak 5524 will supply a relatively constant air flow that does not appreciably increase as additional negative pressure is applied to the system 5501. In embodiments of the suction adapter 5500 where the air flow through the air leak 5524 increases as additional negative pressure is applied, preferably this increased air flow will be minimized and not increase in proportion to the negative pressure applied thereto.

The filter 5525 provided in the controlled air leak 5524 in certain embodiments may be useful in a system 5501 for use with more ambulatory and active patients. For example, a chemically-resistant filter may permit a patient to bathe or shower without damaging the filter's functionality when reconnected to a source of negative pressure. Any occlusion or fluid blocking the air leak 5524 could then be cleared by, for example, wiping off the filter 5525 or re-applying negative pressure to the suction adapter 5500. Such a system would also have the advantage that the system 5501 and any assorted wound dressing materials, if present, would not need to be removed and then re-applied should a patient need to be disconnected from the source of negative pressure, for example incidental to bathing. This would entail significant advantages in improving the cost-effectiveness and ease of use of the present treatment system.

The suction adapter 5500 may be constructed so as to provide a consistent fluid flow even if the suction adapter 5500 is kinked or weighted down. For example, in use on a patient, the bridge portion 5502 may become folded over itself, or else the patient may roll over, thus placing his or her weight over at least a portion of the suction adapter 5500. Typically, prior art dressings and fluidic connectors become blocked or ineffective in such situations and in some cases may contribute to complications such as pressure ulcers. Here, however, certain embodiments provide for improved blockage resistance if kinked or weighed down. By employing channel layers 5512 and 5516 as described above, and by employing a foam channel layer 5512 and a fabric channel layer 5516, the suction adapter 5500 may be able to maintain a flow rate through the air leak 5524 of at least 0.08 L/min, and preferably 0.12 L/min while negative pressure is applied through a source of negative pressure. Further embodiments also provide for the suction adapter 5500 to be able to handle fluid exudate drainage from the wound site through the lower channel 5516 of at least 10 L/day, or 6.9 ml/min. Certain embodiments provide for the suction adapter 5500 to maintain these flow rates with a weight, for example a 12 kg weight, pressing down on the bridge portion through a rod with a 1 in. diameter. In some embodiments, these flow rates are also maintained while the bridge portion 5502 is kinked over itself with the same weight, or for example with a 4.75 kg weight placed directly on the folded region. It is preferable that the suction adapter 5500 be able to withstand being folded or kinked over even during an extended period of time, for example over 40 hours, and not show any degradation in performance (e.g., flow rates) compared to its performance prior to being folded or kinked over. Preferably, embodiments of the suction adapter 5500 are also able to transmit and maintain a negative pressure at the wound that is close to the negative pressure level at the source of negative pressure. For example, an acceptable level of pressure maintained at the wound may be within +-.25 mmHg of the negative pressure set at the source of negative pressure, with this pressure being preferably maintained at this level within 95% of the time that the suction adapter 5500 has negative pressure applied to it. Acceptable pressure levels may include pressure ranges between 40-120 mmHg, although levels of 200 mmHg have successfully been used.

With additional reference to Figs. 4A-5B and 6, the suction adapter 5500 may comprise an applicator 5520 designed for placement over a wound site. The applicator 5520 may comprise a flexible layer 5550, for example polyethylene or polyurethane, with a layer of adhesive on its lower (wound-facing) side. Optionally, a protective release layer 5529 may be placed on the adhesive layer, which is removable before use. In some embodiments, a more rigid removable backing layer 5552 may be provided on the upper side of the applicator 5520 to facilitate handling of the applicator 5520 due to the flexibility of the layer 5550. The applicator 5520 preferably comprises an attachment point for the bridge 5502 at the distal end 5505, for example using a section of double-sided adhesive tape 5528. The double-sided adhesive tape 5528 may be protected by an additional protective release layer, which is removed prior to adhering the bridge 5502 to the applicator 5520. It will be understood that different attachment methods are also contemplated, for example heat sealing, welding, or suitable adhesives. Some embodiments may also permit the manufacture of the bridge 5502 and the applicator 5520 as a single unit that does not require separate attachment means. The applicator 5520 preferably comprises at least one aperture 5526 through itself and designed to be placed over a wound site, and which can serve to fluidically connect the wound site to the source of negative pressure and to the air leak while also serving as a conduit to draw out wound exudate from the wound site.

In use, and with reference to Figs. 4A-B, the system 5501 may be used in a similar fashion to the other embodiments previously disclosed herein, such as the system 100 described in relation to Fig. 1. A wound site 5530 is preferably cleaned and prepared in a suitable fashion, and a wound packing material, if necessary, placed into the wound site, followed by a drape 5531. An aperture 5535 through the drape to the wound site is then created, although some embodiments may have a pre-made aperture 5535. Subsequently, an operator may situate the applicator portion 5520 over the aperture 5535. After removing the backing layer 5529 (if present) from the adhesive layer on the underside of the applicator portion 5520, the applicator is sealed to the drape 5531, and the backing layer 5552 (if present) is also removed from the applicator portion 5520. A fluidic conduit such as a tube 5540 may then be connected to the connector 5504. The tube 5540 may also be connected to connector 5504 prior to applying the applicator to the wound site. The fluidic conduit is connected to a source of negative pressure 5534, preferably with a container suitable for containing wound exudate interposed therebetween. The application of negative pressure may then be effectuated to the wound site 5530 until the wound site progresses to a desired level of healing.

During use of the system 5501, wound exudate from the wound site 5530 may be drawn by the negative pressure through the lower channel layer 5516. The air leak 5524 allows air to pass through the upper channel layer 5512 into the apertures through the distal ends of the layers 5512, 5514, 5516 and 5518. The negative pressure draws air passing through the upper channel layer into the lower channel layer 5516 back toward the source of negative pressure or pump. In some embodiments, the controlled air leak 5524 provides a constant flow of air through the suction adapter 5500, which then may be used to determine whether blockage or leakage is present. Causes of blockage can include, for example, situations where the lower channel 5516 becomes occluded with wound debris. Leakage causes can include, for example, improper sealing of the drape over the wound site, or physical damage to the suction adapter 5500 leading to excess air leaking into the system. The blockage or leakage may be determined, in certain embodiments, by measuring the speed of the pump while the pump works to maintain a constant negative pressure. Pump speed may also be measured indirectly by measuring the amount of voltage or signal sent to the pump.

### Multi-Layered Wound Dressings for NPWT

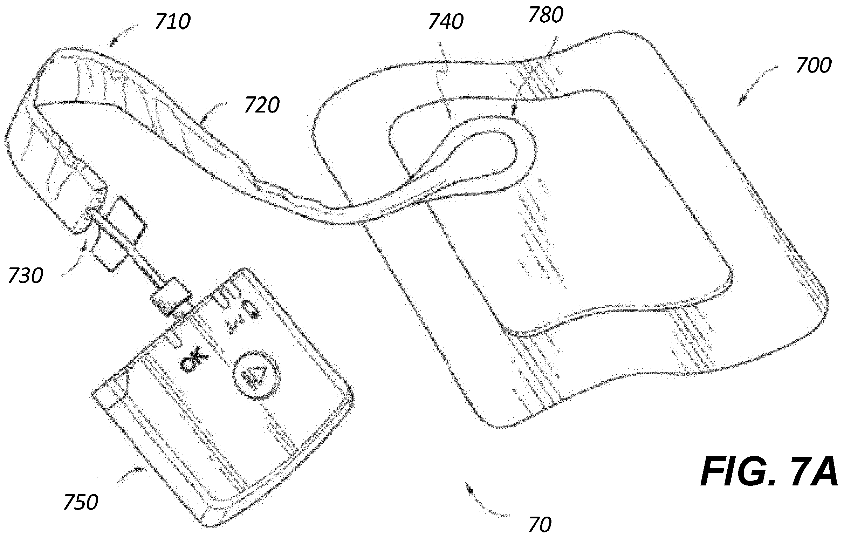

Fig. 7A illustrates an embodiment of a negative pressure wound treatment system 70 employing a wound dressing 700 in conjunction with a fluidic connector 710. Additional examples related to negative pressure wound treatment comprising a wound dressing in combination with a pump as described herein may also be used in combination or in addition to those described in US Patent No. 9,061,095. Here, the fluidic connector 710 may comprise an elongate conduit, more preferably a bridge 720 having a proximal end 730 and a distal end 740, and an applicator 780 at the distal end 740 of the bridge 720. The system 70 may include a source of negative pressure such as a pump or negative pressure unit 750 capable of supplying negative pressure. The pump may comprise a canister or other container for the storage of wound exudates and other fluids that may be removed from the wound. A canister or container may also be provided separate from the pump. In some embodiments, the pump 750 can be a canisterless pump such as the PICO^{™} pump, as sold by Smith & Nephew. The pump 750 may be connected to the bridge 720 via a tube, or the pump 750 may be connected directly to the bridge 720. In use, the dressing 700 is placed over a suitably-prepared wound, which may in some cases be filled with a wound packing material such as foam or gauze as described above. The applicator 780 of the fluidic connector 710 has a sealing surface that is placed over an aperture in the dressing 700 and is sealed to the top surface of the dressing 700. Either before, during, or after connection of the fluidic connector 710 to the dressing 700, the pump 750 is connected via the tube to the coupling 760, or is connected directly to the bridge 720. The pump is then activated, thereby supplying negative pressure to the wound. Application of negative pressure may be applied until a desired level of healing of the wound is achieved.

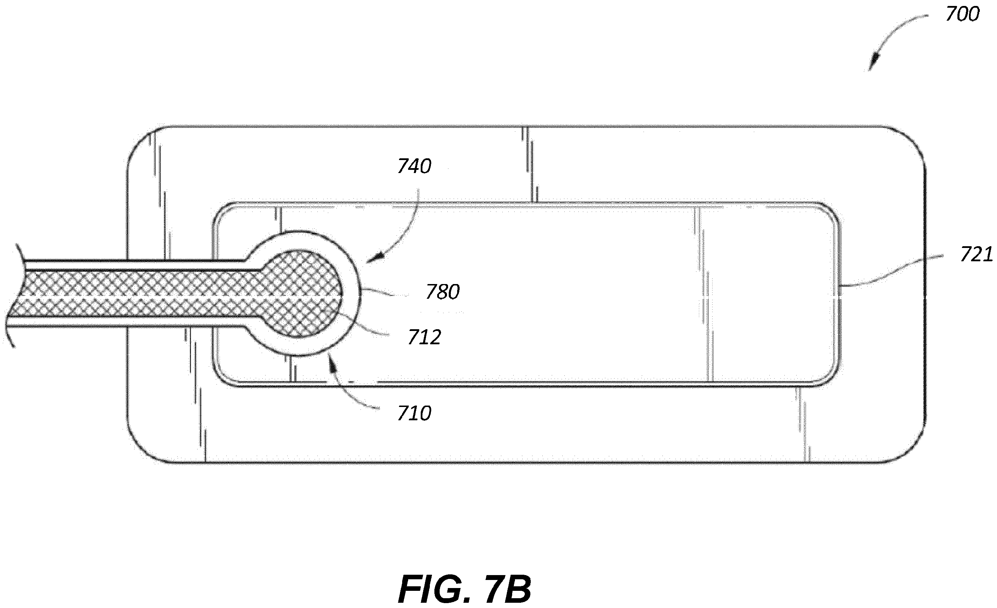

As shown in Fig. 7B, the fluidic connector 710 preferably comprises an enlarged distal end, or head 740 that is in fluidic communication with the dressing 700 as will be described in further detail below. In one embodiment, the enlarged distal end has a round or circular shape. The head 740 is illustrated here as being positioned near an edge of the dressing 700, but may also be positioned at any location on the dressing. For example, some embodiments may provide for a centrally or off-centered location not on or near an edge or corner of the dressing 700. In some embodiments, the dressing 70 may comprise two or more fluidic connectors 710, each comprising one or more heads 740, in fluidic communication therewith. In a preferred embodiment, the head 740 may measure 30mm along its widest edge. The head 740 forms at least in part the applicator 780, described above, that is configured to seal against a top surface of the wound dressing.

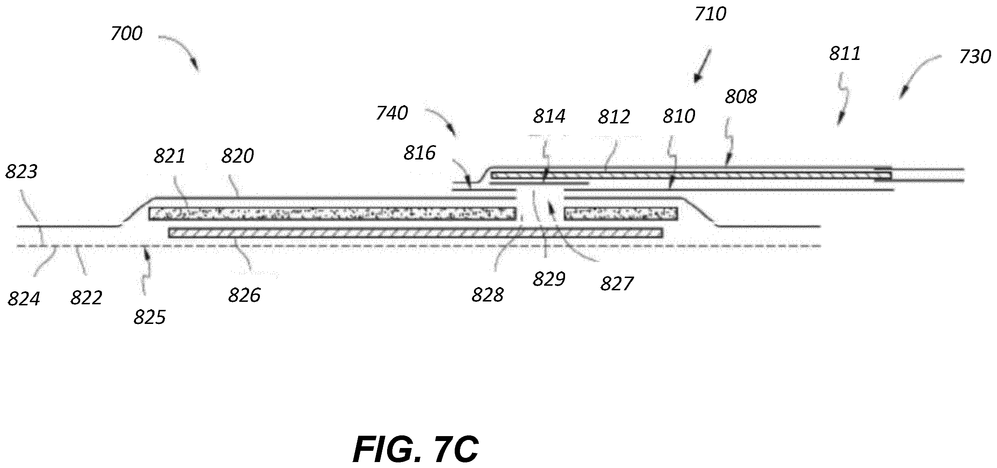

Fig. 7C illustrates a cross-section through a wound dressing 700 similar to the wound dressing 70 as described in International Patent Publication WO2013175306 A2, along with fluidic connector 710. The wound dressing 700, which can alternatively be any wound dressing embodiment disclosed herein or any combination of features of any number of wound dressing embodiments disclosed herein, can be located over a wound site to be treated. The dressing 700 may be placed as to form a sealed cavity over the wound site. In a preferred embodiment, the dressing 700 comprises a top or cover layer, or backing layer 820 attached to an optional wound contact layer 822, both of which are described in greater detail below. These two layers 820, 822 are preferably joined or sealed together so as to define an interior space or chamber. This interior space or chamber may comprise additional structures that may be adapted to distribute or transmit negative pressure, store wound exudate and other fluids removed from the wound, and other functions which will be explained in greater detail below. Examples of such structures, described below, include a transmission layer 826 and an absorbent layer 821.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in Fig. 7C, the wound contact layer 822 can be a polyurethane layer or polyethylene layer or other flexible layer which is perforated, for example via a hot pin process, laser ablation process, ultrasound process or in some other way or otherwise made permeable to liquid and gas. The wound contact layer 822 has a lower surface 824 and an upper surface 823. The perforations 825 preferably comprise through holes in the wound contact layer 822 which enable fluid to flow through the layer 822. The wound contact layer 822 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 822 may help maintain the integrity of the entire dressing 700 while also creating an air tight seal around the absorbent pad in order to maintain negative pressure at the wound.

Some embodiments of the wound contact layer 822 may also act as a carrier for an optional lower and upper adhesive layer (not shown). For example, a lower pressure sensitive adhesive may be provided on the lower surface 824 of the wound dressing 700 whilst an upper pressure sensitive adhesive layer may be provided on the upper surface 823 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 700 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A transmission layer 826 can be located above the wound contact layer 822. In some embodiments, the transmission layer can be a porous material. As used herein the transmission layer can be referred to as a spacer layer and the terms can be used interchangeably to refer to the same component described herein. This transmission layer 726 allows transmission of fluid including liquid and gas away from a wound site into upper layers of the wound dressing. In particular, the transmission layer 826 preferably ensures that an open-air channel can be maintained to communicate negative pressure over the wound area even when the absorbent layer has absorbed substantial amounts of exudates. The layer 826 should preferably remain open under the typical pressures that will be applied during negative pressure wound therapy as described above, so that the whole wound site sees an equalized negative pressure. The layer 826 may be formed of a material having a three-dimensional structure. For example, a knitted or woven spacer fabric (for example Baltex 7970 weft knitted polyester) or a non-woven fabric could be used. The three-dimensional material can comprise a 3D spacer fabric material similar to the material described in International Publication WO 2013/175306 A2 and International Publication WO2014/020440.

In certain embodiments, the wound dressing 700 may incorporate or comprise a multi-care WCL as described herein this section or elsewhere in the specification. One of skill in the art will understand that the wound dressing 700 may incorporate any of the multi-care WCLs disclosed herein this section or elsewhere in the specification. One of skill in the art will also understand that the multi-care WCL may be incorporated as a whole component layer or a part of a component layer. In some embodiments, the multi-care WCL layer may be provided below the transmission layer 826. In some embodiments, the multi-care WCL layer may be provided above the wound contact layer 822. In some embodiments, the multi-care WCL layer may replace the transmission layer 826, such that the multi-care WCL layer is provided between an absorbent layer 221 (described further below) and the wound contact layer 822. In some embodiments, the multi-care WCL layer can supplement or replace the absorbent layer 821. In some embodiments, the wound dressing 700 does not have the wound contact layer 822, and the multi-care WCL layer may be the lowermost layer of the wound dressing 700. The multi-care WCL may have same or substantially similar size and shape with the transmission layer 826 and/or the absorbent layer 821.

The multi-care WCL layer may be constructed to be flexible but stiff enough to withstand negative pressure, such that the multi-care WCL is not collapsed excessively and thereby transmits negative pressure sufficiently to the wound when negative pressure is supplied to the wound dressing 700. The multi-care WCL layer may be constructed to include sufficient number or size of pores to enable transmission of negative pressure through it. Further, the multi-care WCL layer may have suitable thickness to transmit enough negative pressure to the wound. For example, the multi-care WCL layer may have a thickness of 1 mm to 10 mm, or 1 mm to 7 mm, or 1.5 mm to 7 mm, or 1.5 mm to 4 mm, or 2 mm to 3 mm. In some embodiments, the multi-care WCL may have a thickness of approximately 2 mm.

In some embodiments, the layer 821 of absorbent material is provided above the transmission layer 826. The absorbent material, which can comprise a foam or non-woven natural or synthetic material, and which may optionally comprise a super-absorbent material, forms a reservoir for fluid, particularly liquid, removed from the wound site. In some embodiments, the layer 821 may also aid in drawing fluids towards the backing layer 220.

The material of the absorbent layer 821 may also prevent liquid collected in the wound dressing 700 from flowing freely within the dressing, and preferably acts so as to contain any liquid collected within the dressing. The absorbent layer 821 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 821 may typically be manufactured from ALLEVYN^{™} foam, Freudenberg 114-224-4 or Chem-Posite^{™}11C-450. In some embodiments, the absorbent layer 821 may comprise a composite comprising superabsorbent powder, fibrous material such as cellulose, and bonding fibers. In a preferred embodiment, the composite is an air-laid, thermally-bonded composite.

In some embodiments, the absorbent layer 821 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 827 is preferably provided in the backing layer 820 to allow a negative pressure to be applied to the dressing 700. The fluidic connector 710 is preferably attached or sealed to the top of the backing layer 820 over the orifice 827 made into the dressing 700, and communicates negative pressure through the orifice 827. A length of tubing may be coupled at a first end to the fluidic connector 710 and at a second end to a pump unit (not shown) to allow fluids to be pumped out of the dressing. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 710 may be adhered and sealed to the backing layer 820 using an adhesive such as an acrylic, cyanoacrylate, epoxy, UV curable or hot melt adhesive. The fluidic connector 710 may be formed from a soft polymer, for example a polyethylene, a polyvinyl chloride, a silicone or polyurethane having a hardness of 30 to 90 on the Shore A scale. In some embodiments, the fluidic connector 710 may be made from a soft or conformable material.

Optionally, the absorbent layer 821 includes at least one through hole 828 located so as to underlie the fluidic connector 710. The through hole 828 may in some embodiments be the same size as the opening 827 in the backing layer, or may be bigger or smaller. As illustrated in Figure 2C a single through hole can be used to produce an opening underlying the fluidic connector 710. It will be appreciated that multiple openings could alternatively be utilized. Additionally, should more than one port be utilized according to certain embodiments of the present disclosure one or multiple openings may be made in the absorbent layer in registration with each respective fluidic connector. Although not essential to certain embodiments of the present disclosure the use of through holes in the super-absorbent layer may provide a fluid flow pathway which remains unblocked in particular when the absorbent layer is near saturation.

The aperture or through-hole 828 is preferably provided in the absorbent layer 821 beneath the orifice 827 such that the orifice is connected directly to the transmission layer 826 as illustrated in Fig. 2C. This allows the negative pressure applied to the fluidic connector 710 to be communicated to the transmission layer 826 without passing through the absorbent layer 821. This ensures that the negative pressure applied to the wound site is not inhibited by the absorbent layer as it absorbs wound exudates. In other embodiments, no aperture may be provided in the absorbent layer 821, or alternatively a plurality of apertures underlying the orifice 827 may be provided. In further alternative embodiments, additional layers such as another transmission layer or an obscuring layer such as described with reference to Figs. 6A-6B and in International Patent Publication WO2014/020440, may be provided over the absorbent layer 821 and beneath the backing layer 820.

The backing layer 820 is preferably gas impermeable, but moisture vapor permeable, and can extend across the width of the wound dressing 700. The backing layer 820, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way, an effective chamber is made between the backing layer 820 and a wound site where a negative pressure can be established. The backing layer 820 is preferably sealed to the wound contact layer 822 in a border region around the circumference of the dressing, ensuring that no air is drawn in through the border area, for example via adhesive or welding techniques. The backing layer 820 protects the wound from external bacterial contamination (bacterial barrier) and allows liquid from wound exudates to be transferred through the layer and evaporated from the film outer surface. The backing layer 820 preferably comprises two layers; a polyurethane film and an adhesive pattern spread onto the film. The polyurethane film is preferably moisture vapor permeable and may be manufactured from a material that has an increased water transmission rate when wet. In some embodiments, the moisture vapor permeability of the backing layer increases when the backing layer becomes wet. The moisture vapor permeability of the wet backing layer may be up to about ten times more than the moisture vapor permeability of the dry backing layer.

The absorbent layer 821 may be of a greater area than the transmission layer 826, such that the absorbent layer overlaps the edges of the transmission layer 826, thereby ensuring that the transmission layer does not contact the backing layer 820. This provides an outer channel of the absorbent layer 821 that is in direct contact with the wound contact layer 822, which aids more rapid absorption of exudates to the absorbent layer. Furthermore, this outer channel ensures that no liquid is able to pool around the circumference of the wound cavity, which may otherwise seep through the seal around the perimeter of the dressing leading to the formation of leaks. As illustrated in Fig. 7C, the absorbent layer 821 may define a smaller perimeter than that of the backing layer 820, such that a boundary or border region is defined between the edge of the absorbent layer 821 and the edge of the backing layer 820.

As shown in Fig. 7C, one embodiment of the wound dressing 700 comprises an aperture 828 in the absorbent layer 821 situated underneath the fluidic connector 710. In use, for example when negative pressure is applied to the dressing 700, a wound facing portion of the fluidic connector may thus come into contact with the transmission layer 826, which can thus aid in transmitting negative pressure to the wound site even when the absorbent layer 821 is filled with wound fluids. Some embodiments may have the backing layer 820 be at least partly adhered to the transmission layer 826. In some embodiments, the aperture 828 is at least 1-2 mm larger than the diameter of the wound facing portion of the fluidic connector 710, or the orifice 827.

In particular for embodiments with a single fluidic connector 710 and through hole, it may be preferable for the fluidic connector 710 and through hole to be located in an off-center position as illustrated in Fig. 7B. Such a location may permit the dressing 700 to be positioned onto a patient such that the fluidic connector 710 is raised in relation to the remainder of the dressing 700. So positioned, the fluidic connector 710 and the filter 814 may be less likely to come into contact with wound fluids that could prematurely occlude the filter 814 so as to impair the transmission of negative pressure to the wound site.

Similar to the embodiments of wound dressings described above, some wound dressings comprise a perforated wound contact layer with silicone adhesive on the skin-contact face and acrylic adhesive on the reverse. In some embodiments, the wound contact layer may be constructed from polyurethane, polyethylene or polyester. Above this bordered layer sits a transmission layer. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can be a high moisture vapor transmission rate (MVTR) film, pattern coated with acrylic adhesive. The high MVTR film and wound contact layer encapsulate the transmission layer and absorbent layer, creating a perimeter border of approximately 20 mm. The backing layer can have a 10 mm aperture that overlies the aperture in the absorbent layer. Above the hole can be bonded a fluidic connector that comprises a liquid-impermeable, gas-permeable semi-permeable membrane (SPM) or filter that overlies the aforementioned apertures.

### Multilayered Wound Dressing with an Integrated Source of Negative Pressure

In some embodiments, a source of negative pressure (such as a pump) and some or all other components of the TNP system, such as power source(s), sensor(s), connector(s), user interface component(s) (such as button(s), switch(es), speaker(s), screen(s), etc.) and the like, can be integral with the wound dressing. Additionally, some embodiments related to wound treatment comprising a wound dressing described herein may also be used in combination or in addition to those described in International Application WO 2016/174048 and International Patent Application PCT/EP2017/055225, filed on March 6, 2017, entitled "WOUND TREATMENT APPARATUSES AND METHODS WITH NEGATIVE PRESSURE SOURCE INTEGRATED INTO THE WOUND DRESSING,", including further details relating to embodiments of wound dressings, the wound dressing components and principles, and the materials used for the wound dressings and wound dressing components.

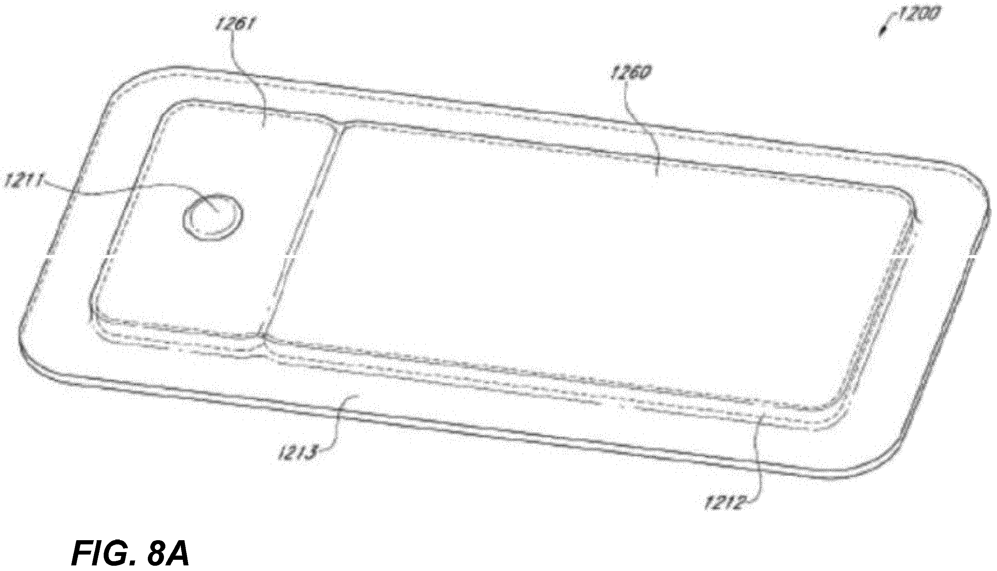

In some embodiments, the pump and/or other electronic components can be configured to be positioned adjacent to or next to the absorbent and/or transmission layers in the wound dressing so that the pump and/or other electronic components are still part of a single apparatus to be applied to a patient with the pump and/or other electronics positioned away from the wound site. Fig. 8A illustrates a wound dressing incorporating the source of negative pressure and/or other electronic components within the wound dressing. Fig. 8A illustrates a wound dressing 1200 with the pump and/or other electronics positioned away from the wound site. The wound dressing can include an electronics area 1261 and an absorbent area 1260. The dressing can comprise a wound contact layer (not shown) and a moisture vapor permeable film or cover layer 1213 positioned above the contact layer and other layers of the dressing. The wound dressing layers and components of the electronics area as well as the absorbent area can be covered by one continuous cover layer 1213 as shown in Fig. 8A.

The electronics area 1261 can include a source of negative pressure (such as a pump) and some or all other components of the TNP system, such as power source(s), sensor(s), connector(s), user interface component(s) (such as button(s), switch(es), speaker(s), screen(s), etc.) and the like, that can be integral with the wound dressing. For example, the electronics area 1261 can include a button or switch 1211 as shown in Fig. 8A. The button or switch 1211 can be used for operating the pump (e.g., turning the pump on/off).

The absorbent area 1260 can include an absorbent material 1212 and can be positioned over the wound site. The electronics area 1261 can be positioned away from the wound site, such as by being located off to the side from the absorbent area 1260. The electronics area 1261 can be positioned adjacent to and in fluid communication with the absorbent area 1260 as shown in Fig. 8A. In some embodiments, each of the electronics area 1261 and absorbent area 1260 may be rectangular in shape and positioned adjacent to one another.

In some embodiments, additional layers of dressing material can be included in the electronics area 1261, the absorbent area 1260, or both areas. In some embodiments, the dressing can comprise one or more spacer or transmission layers and/or one or more absorbent layers positioned above the contact layer and below the wound cover layer 1213 of the dressing.

The dressing can comprise a multi-care WCL, as described above or described elsewhere herein, a transmission layer (not shown), an absorbent layer 1212 over the transmission layer, a moisture vapor permeable film or cover layer 1213 positioned above the wound contact layer, transmission layer, absorbent layer, or other layers of the dressing. The wound contact layer can be configured to be in contact with the wound. The wound contact layer can include an adhesive on the patient facing side for securing the dressing to the surrounding skin or on the top side for securing the wound contact layer to a cover layer or other layer of the dressing. In operation, the wound contact layer can be configured to provide unidirectional flow so as to facilitate removal of exudate from the wound while blocking or substantially preventing exudate from returning to the wound. The one or more transmission layers assist in distributing negative pressure over the wound site and facilitating transport of wound exudate and fluids into the wound dressing. In some embodiments, the transmission layer can be formed at least partially from a three-dimensional (3D) fabric. Further, an absorbent layer (such as layer 1212) for absorbing and retaining exudate aspirated from the wound can be utilized. In some embodiments, a superabsorbent material can be used in the absorbent layer 1212. In some embodiments, the absorbent includes a shaped form of a superabsorber layer. The wound dressing layers of the electronics area and the absorbent layer can be covered by one continuous cover layer 1213. In some embodiments, the cover layer can include a moisture vapor permeable material that prevents liquid exudate removed from the wound and other liquids from passing through, while allowing gases through.

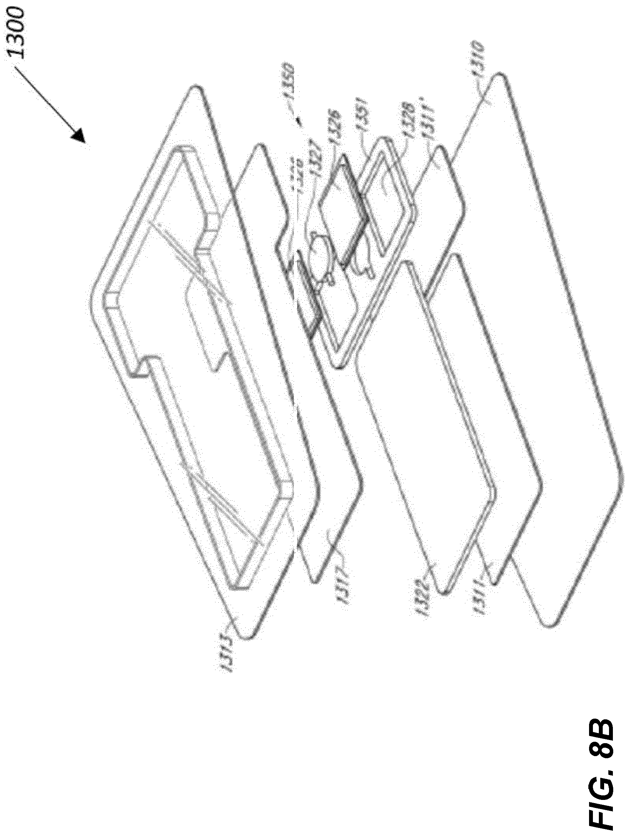

Fig. 8B illustrates an embodiment of layers of a wound dressing with the pump and electronic components offset from the absorbent area of the dressing. As illustrated in Fig. 8B, the dressing can include a wound contact layer 1310 for placing in contact with the wound. Lower spacer or transmission layers 1311 and 1311' are provided above the wound contact layer 1310. In some embodiments, the transmission layer 1311 can be a separate layer from spacer layer 1311' as shown in Fig. 8B. The lower transmission layers 1311 and/or 1311' can assist in distributing pressure evenly to the wound surface and/or wicking fluid away from the wound. An absorbent layer 1322 can be positioned above the lower transmission layer 1311.

In embodiments, a dressing layer 1351 can include cutouts or recesses 1328 for embedding the electronic components 1350 within the layer 1351. In some embodiments, the cutouts or recesses 1328 can be sized and shaped to embed a pump 1327, power source 1326, and/or other electronic components. In some embodiments, the layer 1351 can include multiple spacer or transmission layers stacked together. In some embodiments, the layer 1351 can include multiple spacer or transmission layers pieced together to surround the electronic components 1350. An upper transmission layer 1317 can be provided above the absorbent layer 1322, layer 1351, and/or electronic components 1350.

The wound dressing 1200, 1300 may incorporate or comprise a multi-care WCL as described herein this section or elsewhere in the specification. One of skill in the art will understand that the wound dressing 1200, 1300 may incorporate any of the multi-care WCLs disclosed herein this section or elsewhere in the specification. In some embodiments, the multi-care WCL layer may be provided below the transmission layer 1311. In some embodiments, the multi-care WCL layer may be provided below the wound contact layer 1310. In some embodiments, the multi-care WCL layer may replace the transmission layer 1311, 1311' such that the multi-care WCL layer is provided between an absorbent layer 1322 and the wound contact layer 1310. In some embodiments, the multi-care WCL layer can supplement or replace the absorbent layer 1212, 1322. In some embodiments, the multi-care WCL layer may be the lowermost layer of the wound dressing. The multi-care WCL layer may have same or substantially similar size and shape with the transmission layers and/or the absorbent layers described herein this section or elsewhere in the specification.

The multi-care WCL layer may be constructed to be flexible but stiff enough to withstand negative pressure, such that the multi-care WCL layer is not collapsed excessively and thereby transmits negative pressure sufficiently to the wound when negative pressure is supplied to the wound dressing 1200. The multi-care WCL layer may be constructed to include sufficient number or size of pores to enable transmission of negative pressure through it. Further, the multi-care WCL layer may have suitable thickness to transmit enough negative pressure to the wound. For example, the multi-care WCL layer may have a thickness of 1 mm to 10 mm, 1 mm to 7 mm, 1.5 to 7 mm, 1.5 mm to 4 mm, or 2 mm to 3 mm. In some embodiments, the multi-care WCL layer may have a thickness of approximately 2 mm.

A cover layer or backing layer 1313 can be positioned over the upper transmission layer 1317. The backing layer 1313 can form a seal to the wound contact layer 1310 at a perimeter region enclosing the transmission layers 1311, 1311', and 1317, the absorbent layer 1322, layer 1351, and electronic components 1350. In some embodiments, the backing layer 1313 can be a flexible sheet of material that forms and molds around the dressing components when they are applied to the wound. In other embodiments, the backing layer 1313 can be a material that is preformed or premolded to fit around the dressing components as shown in Fig. 8B.

### Fixation strip

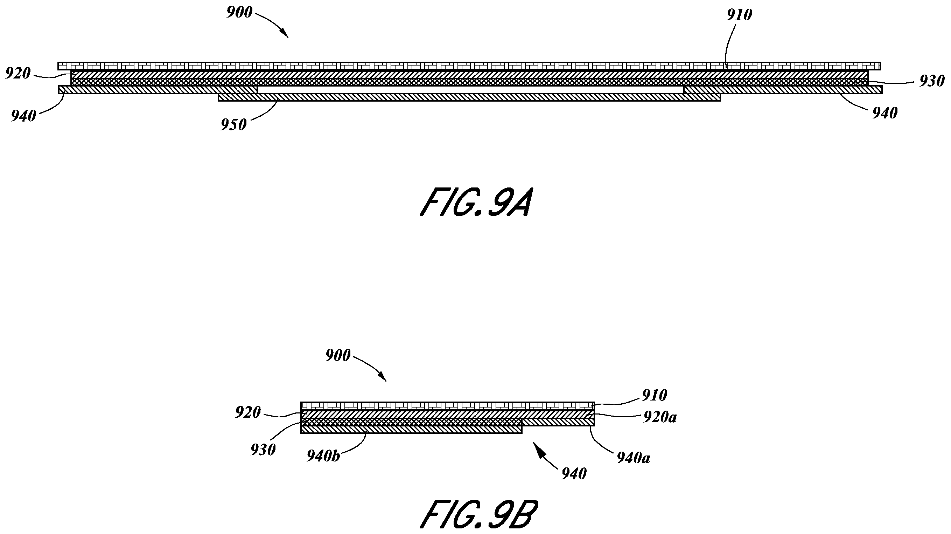

Figs. 9A-9B illustrate an embodiment of a fixation strip 900 that can be used with any of the system configurations, devices, apparatuses, and/or dressings disclosed herein this section or elsewhere in the specification, such as in Figs 1-8B, 10A-10B, 11A-11B, or 13A-13B. For example, such a fixation strip could be used with the dressings 700, 1200 and/or drapes 5531 described above or the drape 1400 described below. Fig. 9A illustrates a cross-sectional side view of the fixation strip 900 along the length of the strip 900 and Fig. 9B illustrates a cross-sectional side view of the fixation strip 900 along the width of the strip 900.

As shown in Figs. 9A-9B, a fixation strip 900 may comprise a plurality of layers. The plurality of layers may include a top layer 910, an adhering layer 920, a wound sealing layer 930, and one or more protective liners 940, 950. The adhering layer 920 may, in some embodiments, comprise a flexible film material provided with a pressure-sensitive adhesive (e.g., an acrylic adhesive) on a lower surface of the adhering layer 920. In some embodiments, the pressure-sensitive adhesive of the adhering layer 920 may extend the entire width of the fixation strip. One of skill in the art will understand that the adhering layer may be constructed from any suitable material disclosed herein, such as the cover layers and/or drapes disclosed herein. The adhering layer 920 may be thin in some areas of the fixation strip 900, thereby allowing for the fixation strip 900 to be folded or self-adhere as needed. In some embodiments, the adhering layer 920 may be overlain by a top layer 910 on an upper surface of the adhering layer 920.

In certain examples, the top layer 910 may have the same length and width as the adhering layer 920. The top layer 910 may also have different dimensions from the adhering layer 920, for example the top layer 910 may be longer or shorter than the adhering layer 920 in the width and/or length. In some configurations, as shown in Fig. 9A, the top layer 910 can have a greater length than the adhering layer 920. In some configurations, as shown in Fig. 9B, the top layer 910 can have the same width as the adhering layer 920.

In some configurations, the adhering layer 920 may have the same length and width as the wound sealing layer 930. The adhering layer 920 may also have different dimensions from the wound sealing layer 930, for example the adhering layer 920 may be longer or shorter than the wound sealing layer 930 in the width and/or length. In some configurations, as shown in Fig. 9B, the adhering layer 920 may have a width that is the same as or similar to a width of the wound sealing layer 930. In some embodiments, as shown in Fig. 9B, the adhering layer 920 may have a greater width than the wound sealing layer 930 such that the adhering layer 920 may extend beyond the wound sealing layer 930 on a side 920a of the fixation strip 900. The extended side 920a may be configured to secure the fixation strip 900 to a wound dressing. In certain embodiments, the adhering layer 920 may extend beyond the wound sealing layer 930 on one, two, three, or four sides of the layer 930. As one of skill in the art will understand, by extending the adhering layer 920 beyond the wound sealing layer 930, the adhering layer 920 may be placed into contact with and/or adhere to, a different surface than the wound sealing layer 930. For example, the wound sealing layer 930 may be placed over a wound or the area surrounding the wound, such as the periwound area, while the adhering layer 920 is placed over a wound dressing such as disclosed herein. Such an arrangement allows for sealing of the wound dressing without exposing the wound or the skin to the adhering surface 920, which may be less suitable for skin or wound contact than the wound sealing layer 930.

In certain embodiments, the wound sealing layer 930 may comprise a flexible, biocompatible material, such as silicone or any suitable material disclosed herein. The wound sealing layer 930 may further be constructed in the same manner as any wound contact layer disclosed herein, such as the wound contact layers of Figures 7A-8B. The wound sealing layer 930 can further comprise a silicone adhesive on the skin-contacting surface and acrylic adhesive on the upper surface. In some embodiments, the silicone adhesive may extend along a portion of the entire width of the fixation strip. The wound sealing layer 930 may be entirely constructed from silicone. In some embodiments, the wound sealing layer 930 may be constructed from polyurethane, polyethylene or polyester. This layer 930 can further comprise a plurality of apertures, which can be created, for example, via a hot pin process, laser ablation process, ultrasound process or via another suitable method to be made permeable to liquid and gas. The apertures may comprise through holes in the wound sealing layer 930 which enable fluid to flow through the layer 930. The array of apertures may comprise a shape selected from the group consisting of round, oval, triangular, square, rectangular, hexagonal, octagonal and any other polygonal shape. The wound sealing layer 930 may prevent tissue ingrowth into the other material of the fixation strip 900. The apertures may be small enough to meet this requirement while still allowing fluid to flow there-through. For example, apertures formed as slits or holes having a size ranging from 0.025 mm to 1.2 mm are considered small enough to help prevent tissue ingrowth into the fixation strip 900 while allowing wound exudate to flow into the fixation strip 900. However, in certain embodiments, the apertures may be sized at least 0.5 mm, between 0.5 to 5 mm, or between 1 to 3 mm. The space between two adjacent holes can be in the range between 0.5 to 5 mm, between 0.5 to 3.5 mm, or between 1 to 3 mm. The thickness of the wound sealing layer 930 can be in the range of 1 to 10 mm, or 1 to 7 mm, or preferably 1.5 to 7 mm, or 1.5 to 4 mm, or 2 to 3 mm, or approximately 2 mm. In some configurations, the wound sealing layer 930 may help maintain the integrity of the entire fixation strip 900 while also creating an air tight seal around perimeter of the fixation strip 900 in order to maintain negative pressure at the wound.

In some embodiments, the fixation strip 900 can be manufactured with the adhering layer 920 and the wound sealing layer 930 laminated together via an adhesive layer (not shown). In embodiments, the adhering layer 920 may be directly laminated (e.g., heat laminated) to the wound sealing layer 930, without the need for an adhesive layer.

In certain embodiments, the fixation strip 900 may also be provided with one or more protective liners 940, 950 on the lower, adhesive surface of the fixation strip 900. As one of skill in the art will understand, such protective liners may be the same or similar to the protective release layers disclosed herein and may be constructed in any suitable manner and from any suitable material disclosed herein. In certain embodiments, such liners serve to protect adhesive surfaces during handling and transport prior to use. The one or more protective liners 940, 950 may be configured to protect the adhesive surface of the wound sealing layer 930 and the adhering layer 920. The one or more protective liners 940, 950 can include one or more outer protective liners 940 and/or one or more central protective liners 950. The one or more outer protective liners can include first and second outer protective liners 940 that may be provided at opposite ends of the fixation strip 900, thereby covering opposite ends of the strip 900. For example, one outer protective liner 940 can protect the adhesive surface of the extended side 920a of the adhering layer 920 and the other outer protective liner 940 can protect the adhesive surface of the wound sealing layer 930 on the opposite side of the extended side 920a. In some configurations, as shown in Fig. 9A, the outer protective liners 940 can protect the adhesive surface of the wound sealing layer 930 on opposite sides of the wound sealing layer 930. A central protective liner 950 may be provided over a central portion of the fixation strip 900. For example, the central protective liner 950 may be positioned between the opposite ends of the fixation strip 900 and partially overlapping with and underlying the outer protective liners 940.

In some embodiments, the outer protective liner 940 may have an outer edge that is positioned beyond the extended side 920a of the adhering layer 920 and the edge of the wound sealing layer 930, and may also include a folded handle that is covered by the central protective liner 950. The folded handles of protective liner 940 are therefore not in direct contact with the adhesive surface of the adhering layer 920 or the wound sealing layer 930, thereby facilitating removal of the outer protective liners 940. Similarly, the portions of the central protective liner 950 overlapping the outer protective liners 940 are not in direct contact with the adhesive surface of the wound sealing layer 930, and are not adhered to the outer protective liners 940, thereby forming handles to facilitate removal of the central protective liner 950.

In some configurations, the protective liners 940, 950 can each include a length less than an entire length of the fixation strip 900 and be positioned lengthwise along the fixation strip 900, as shown in Fig. 9A. In some configurations, each of the protective liners 940, 950 can include a width that extends an entire width of the fixation strip 900. For example, as shown in Fig. 9B, the outer protective liner 940 can extend from the extended side 920a of the adhering layer 920 to the outer edge of the wound sealing layer 930 such that the outer protective liner 940 covers the adhesive surfaces of both the adhering layer 920 and the wound sealing layer 930. In these configurations, the other outer protective liner 940 and the central protective liner 950 can similarly extend from the extend side 920a of the adhering layer 920 to the outer edge of the wound sealing layer 930 to cover the adhesive surfaces of both layer 920, 930. In some configurations, each protective liner 940, 950 can include at least two liners. For example, as shown in Fig. 9B, an outer protective liner 940 can include a first liner 940a and a second liner 940b. Similarly, the other outer protective liner 940 and the central protective liner 950 can include first and second liners that can be configured to removably adhere to the adhesive surface of the extended side 920a of the adhering layer 920 and adhesive surface of the wound sealing layer 930, respectively. In use, the user can remove the first liners and adhere the extended side 920a of the adhering layer 920 to a wound dressing then remove the second liners to adhere the wound sealing layer 930 to the skin of the patient.

The top layer 910 that may be provided on the upper surface of the adhering layer 920 may be configured to releasably attach to the non-adhesive surface of the adhering layer 920, and may comprise a sheet of paper or film with relatively more rigidity than the adhering layer 920. Release tabs (not shown) may be provided on one or both opposite ends of the top layer 910 for ease of removing the top layer 910 from the adhering layer 920. The release tabs may extend outwardly from the adhering layer 920 and top layer 910 to an outer edge aligned with an outer edge of the outer protective liner 940. In some embodiments, graphical and/or numbered instructions for removal of the protective liner and top layer 910 may be provided on one or both of the protective liner and top layer 910. In certain embodiments, there may be a protective liner adhered to the entirety of the adhesive surface of the adhering layer 920.

In some configurations, the fixation strip 900 can be part of a sheet of fixation strips 900. For example, a plurality of fixation strips 900 can be removably attached along the longitudinal sides of each strip 900 to form the sheet. A sheet of fixation strips 900 can include two, three, four, five, six, or more fixation strips 900. Advantageously, a user can remove a suitable number of individual fixation strips 900 from the sheet of fixation strips 900 as the user is applying a wound dressing to a patient.

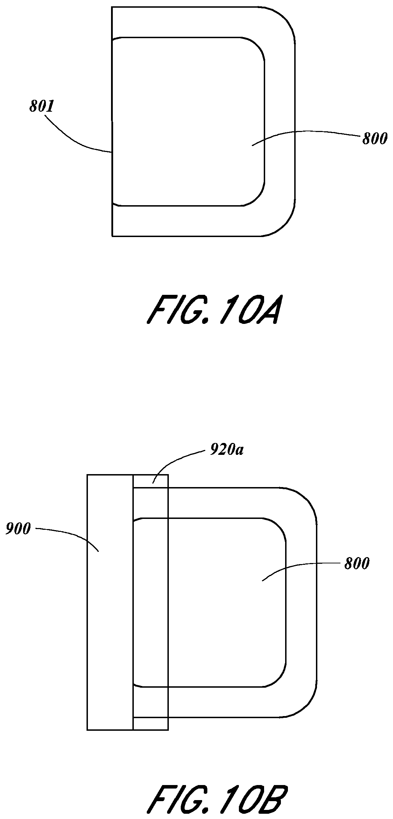

Figs. 10A-10B show a top view of the wound dressing 800 before and after the fixation strip 900 is applied. The wound dressing 800 may be any suitable wound dressing, such as the dressings disclosed in Figs. 7A-8B. As shown in Fig. 10A, the wound dressing 800 may first be cut along one side 801 of the dressing 800. As described above, the extended side 920a of the adhering layer 920 can extend beyond the wound sealing layer 930 such that the extended side 920a of the adhering layer 920 adheres to the upper surface of the dressing 800 such as described above.

To utilize the fixation strips 900 as described above, the central protective liner 950 may be removed using the non-adhered portions of the central protective liner 950, which serve as handles, for exposing a central adhesive surface of the wound sealing layer 930. The adhesive surface may then be applied to skin and/or a dressing or any desired location, or the adhesive surface may be applied after one or both of the outer protective liners 940 is removed. The folded handle of outer protective liners 940 may be grasped to remove the outer protective liners 940, exposing the entirety of the lower adhesive surface of the wound sealing layer 940 and the extended side 920a of the adhering layer 920. The outer edges of the adhesive surface of the wound sealing layer 930 and the extended side 920a of the adhering layer 920 may be placed in a desired location. For example, the extended side 920a of the adhering layer 920 may be placed on a wound dressing. After sealing the fixation strip 900, the release tab or tabs may be used to remove the top layer 910 from the adhering layer 920. This may be repeated with as many fixation strips as are needed. As will be understood by one of skill in the art, fixations strips may be added around the periphery of the dressing as needed to secure and maintain a seal for the application of negative pressure.

In some embodiments, each fixation strip 900 may have a width of 40 mm, or approximately 40 mm, or a width in the range of 20 mm to 80 mm, or approximately 20 mm to 80 mm. The width of the fixation strip 900 can be between approximately 30 mm and 70 mm or approximately 40 mm and 60 mm. Alternatively, a width of less than 20 mm or more than 80 mm can be used. The overall length of each fixation strip 900 may be 250 mm or 300 mm in some embodiments, or approximately 250 mm or approximately 300 mm, or in the range of 100 mm to 400 mm, or approximately 100 mm to approximately 400 mm. The overall length of the fixation strip 900 can be between approximately 150 mm and 350 mm or approximately 200 mm and 300 mm. Alternatively, an overall length of less than 100 mm or more than 400 mm can be used.

The length of the adhering layer 920 and carrier layer 910 may be 280 mm or 330 mm in some embodiments, or approximately 280 mm or approximately 330 mm, or in the range of 90 mm to 380 mm, or approximately 90 mm to approximately 380 mm. The length of the adhering layer 920 and carrier layer 910 can be between approximately 120 mm and 350 mm, approximately 150 mm and 320 mm, approximately 180 mm and 290 mm, approximately 150 mm and 260 mm, or approximately 180 mm and 230 mm. Alternatively, a length of less than 90 mm and more than 380 mm can be used. The length of a central protective liner 950 may be 210 mm or 260 mm in some embodiments, or approximately 210 mm or approximately 260 mm, or may be in the range of 100 mm to 300 mm, or approximately 100 mm to approximately 300 mm. The length of the central protective liner 950 can be between approximately 125 mm and 275 mm, approximately 150 mm and 250 mm or approximately 175 mm and 225 mm. Alternatively, a length of less than 100 mm or more than 300 mm can be used.

The length of the wound sealing layer 940 may be 140 mm or 165 mm in some embodiments, or approximately 140 mm or approximately 165 mm, or in the range of 45 mm to 190 mm, or approximately 45 mm to approximately 190 mm. The length of the wound sealing layer 940 can be between approximately 60 mm and 175 mm, approximately 75 mm and 160 mm, approximately 90 mm and 145 mm, approximately 75 mm and 130 mm, or approximately 90 mm and 115 mm. Alternatively, a length of less than 45 mm and more than 190 mm can be used.

The length of outer protective liners 940 (not including the folded portion) may be 85 mm or 110 mm in some embodiments, or approximately 85 mm or approximately 110 mm, or may be in the range of 50 mm to 200 mm, or approximately 50 mm to approximately 200 mm. The length of the outer protective liners 940 can be between approximately 75 mm and 175 mm or approximately 100 mm and 150 mm. Alternatively, a length of less than 50 mm or more than 200 mm can be used. The length of the folded portion or handle of outer protective liner 940 may be 20 mm plus or minus 5 mm, in some embodiments, or approximately 20 mm plus or minus approximately 5 mm. Alternatively, a length of more than 25 mm can be used. The distance from the outer edge of the folded tab to the outer edge of the central protective liner 950 may be 20 mm plus or minus 5 mm, in some embodiments, or approximately 20 mm plus or minus approximately 5 mm. Alternatively, a distance of more than 25 mm can be used.

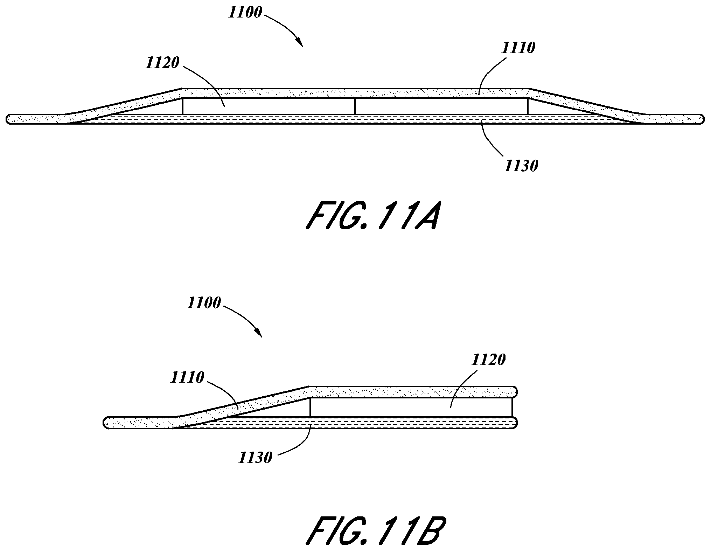

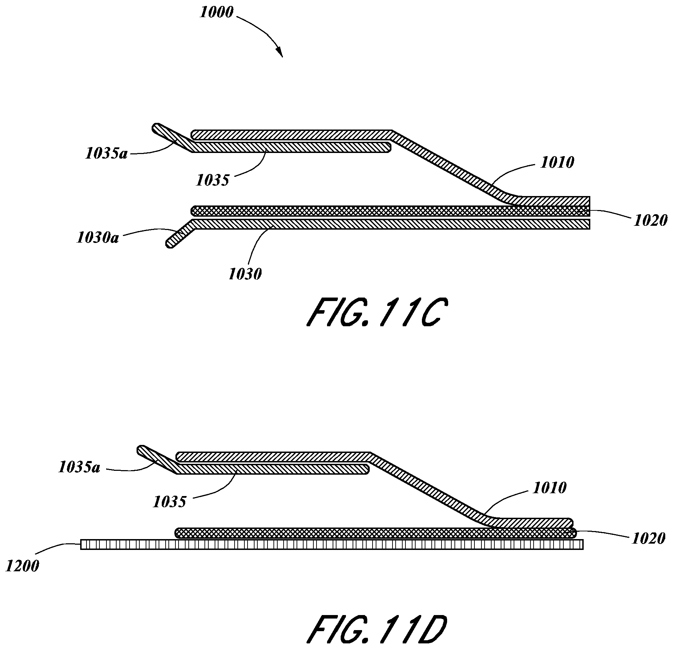

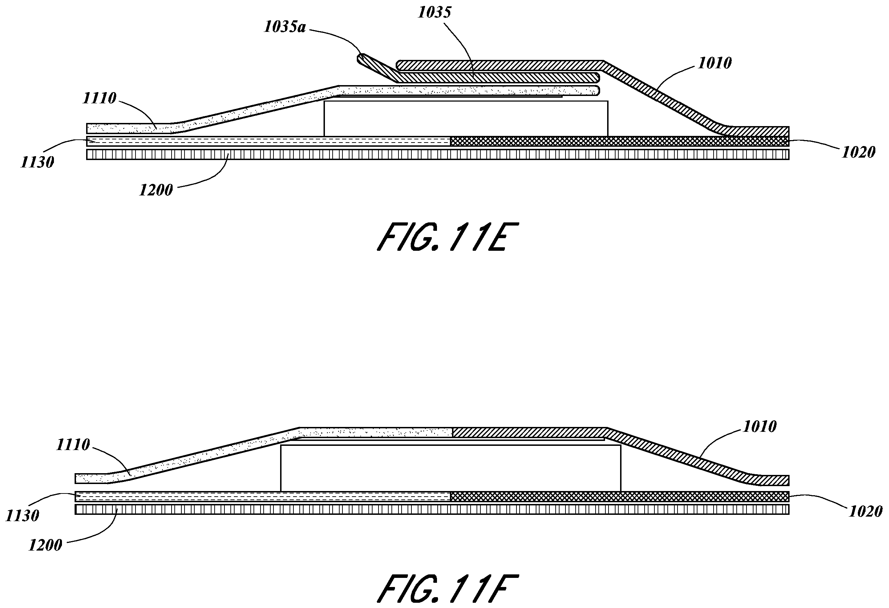

Figs. 11A-F illustrate a method of using an embodiment of a fixation strip 1000. The fixation strip 1000 can be used with any of the system configurations, devices, apparatuses, and/or dressings disclosed herein this section or elsewhere in the specification, such as in Figs 1-8B and 13A-B. For example, such a fixation strip 1000 could be used with drape 5531 or a suitable dressing 700, 1200 as described above. As illustrated in Fig. 11C, the retention strip 1000 may comprise a plurality of layers. In some embodiments, the plurality of layers may include a first protection liner 1030, a wound sealing layer 1020, a second protection liner 1035, and an adhering layer 1010. The adhering layer 1010 may, in some embodiments, comprise a flexible film material provided with a pressure-sensitive adhesive (e.g., an acrylic adhesive) on a lower surface of the adhering layer 1010. In some embodiments, the pressure-sensitive adhesive may extend the entire width of the fixation strip. The adhering layer 1010 may be thin in some areas of the fixation strip 1000 such as described above in relation to Figs. 7-8B. Therefore, the adhering layer 1010 may be provided with a top layer (not shown) on an upper, non-adhesive surface having the same length and width as the adhering layer 1010.

In certain embodiments, the wound sealing layer 1020 may comprise a flexible, biocompatible material, such as silicone. This layer 1020 can further comprise a silicone adhesive on the skin-contacting surface and acrylic adhesive on the upper surface. The silicone adhesive may, in some embodiments, extend a portion of the entire width of the fixation strip. In some embodiments, the wound sealing layer 1020 may be constructed from polyurethane, polyethylene or polyester. This layer 1020 can further comprise a plurality of apertures, which can be created, for example, via a hot pin process, laser ablation process, ultrasound process or via another suitable method to be made permeable to liquid and gas. The apertures may comprise through holes in the wound sealing layer 1020 which enable fluid to flow through the layer 1020. The array of apertures may comprise a shape selected from the group consisting of round, oval, triangular, square, rectangular, hexagonal, octagonal and any other polygonal shape. The wound sealing layer 1020 may prevent tissue ingrowth into the other material of the fixation strip 1000. The apertures may be small enough to meet this requirement while still allowing fluid to flow there-through. For example, apertures formed as slits or holes having a size ranging from 0.025 mm to 1.2 mm are considered small enough to help prevent tissue ingrowth into the fixation strip 1000 while allowing wound exudate to flow into the fixation strip 1000. However, in certain embodiments, the apertures may be sized at least 0.5 mm, between 0.5 to 5 mm, or between 1 to 3 mm. The space between two adjacent holes can be in the range between 0.5 to 5 mm, between 0.5 to 3.5 mm, or between 1 to 3 mm. The thickness of the wound sealing layer 1020 can be in the range of 1 to 10 mm, or 1 to 7 mm, or preferably 1.5 to 7 mm, or 1.5 to 4 mm, or 2 to 3 mm, or approximately 2 mm. In some configurations, the wound sealing layer 1020 may help maintain the integrity of the entire fixation strip 1000 while also creating an air tight seal around perimeter of the fixation strip 1000 in order to maintain negative pressure at the wound.

In certain embodiments, the wound sealing layer 1020 may have a smaller footprint than the top layer 1010. In some embodiments, the fixation strip 1000 can be manufactured with the top layer 1010 and the wound sealing layer 1020 laminated together along one side of the retention strip 1000 via an adhesive layer (not shown). In other embodiments, the top layer 1010 may be directly laminated along one side of the retention strip 1000 (e.g., heat laminated) to the wound sealing layer 1020, without the need for an adhesive layer.

In certain embodiments, the fixation strip 1000 may also be provided with one or more protective liners 1030, 1035 that may be configured to protect the adhesive surface of the adhering layer 1010 or the wound sealing layer 1020. The first protective liner 1030 may be provided over the adhesive surface of the wound sealing layer 1020. In some embodiments, the first protective liner 1030 can cover the entire lower, adhesive surface of the wound sealing layer 1020. The second protective liner 1035 may partially cover the lower, adhesive surface of the adhering layer 1010. As illustrated, the protective liners 1030, 1035 may have an outer edge (shown on the left in Fig. 9C) that is positioned beyond the outer edge of the adhering layer 1010 or the wound sealing layer 1020, and may also include a release handle 1030a, 1035a that is not in direct contact with the adhesive surface of the adhering layer 1010 or the wound sealing layer 1020 to facilitate removal of the protective liners 1030, 1035.

In some configurations, the fixation strip 1000 can be part of a sheet of fixation strips 1000. For example, a plurality of fixation strips 1000 can be removably attached along the longitudinal sides of each strip 1000 to form the sheet. A sheet of fixation strips 1000 can include two, three, four, five, six, or more fixation strips 900. Advantageously, a user can remove a suitable number of individual fixation strips 1000 from the sheet of fixation strips 1000 as the user is applying a wound dressing to a patient.

To utilize the fixation strips 1000 as described above, a dressing 1100 may be cut. The dressing 1100 may comprise a wound sealing layer 1130, an intermediate layer 1120, and a top layer 1110. The first protective liner 1030 may be removed using the release handle 1035 for exposing an adhesive surface of the wound sealing layer 1020. The adhesive surface may then be applied to skin 1200 and/or a dressing or any desired location, or the adhesive surface may be applied after the second contact layer 1035 is removed. The cut wound dressing 1100 may be positioning between the wound sealing layer 1020 and the adhering layer 1010. The release handle 1035 of the second protective liner 1035 may be grasped to remove the second protective liner 1035, exposing the lower adhesive surface of the adhering layer 1010. The adhering layer 1010 may be placed in a desired location. For example, the adhering layer 1010 may be placed on the top layer 1110 of the wound dressing 1100. After sealing the fixation strip 1000, the release tab or tabs may be used to remove the top layer (not shown) from the adhering layer 1010. This may be repeated with as many fixation strips as are needed such as described above.

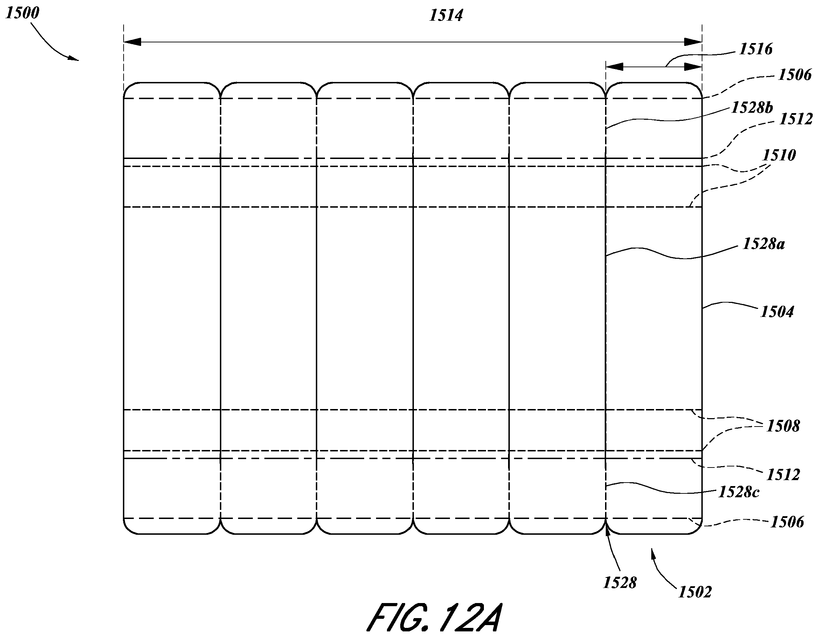

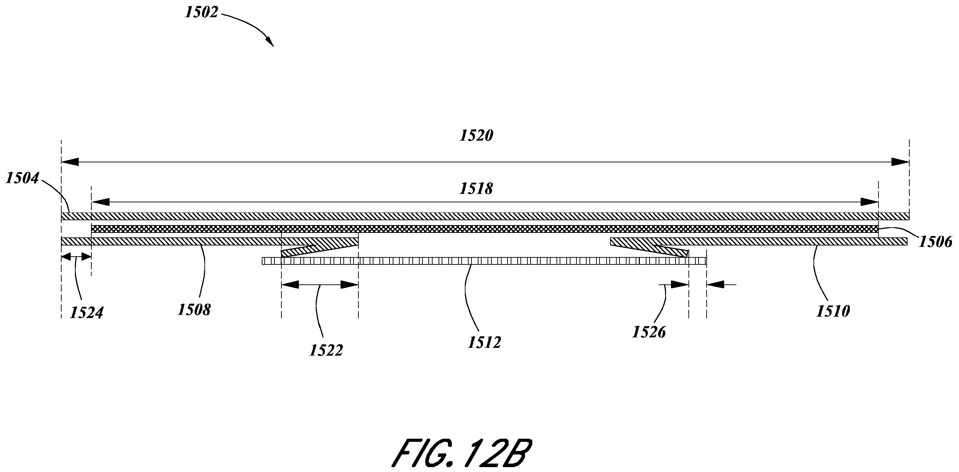

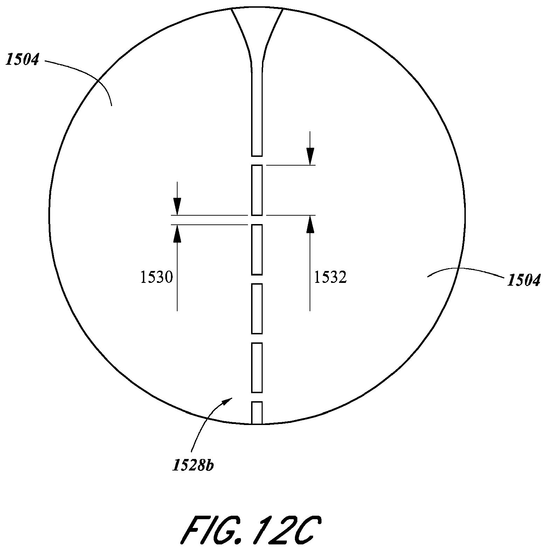

Figs. 12A-12C illustrate a configuration of a fixation strip 1502 that can be used with any of the system configurations, devices, apparatuses, and/or dressings disclosed herein this section or elsewhere in the specification, such as in Figs 1-8B, 10A-10B, 11A-11B, or 13A-13B. Fig. 12A is a top view of a sheet 1500 of a plurality of fixation strips 1502 with the lower layers shown in dashed lines. Fig. 12B is a cross-sectional side view of the fixation strip 1502. Fig. 12C is a close up view of a perforated pattern between each of the plurality of fixation strips 1502 as further described below.

The fixation strip 1502 may have one or more layers. For example, as shown in Fig. 12B, the fixation strip 1502 may have a top layer 1504, an adhering layer 1506, and one or more protective liners 1508, 1510, 1512. The top layer 1504 can be the same as or similar to the top layer 910, 1010, the adhering layer 1506 can be the same as or similar to the adhering layer 920, 1010, and the one or more protective liners 1508, 1510, 1512 can be the same as or similar to the one or more protective liners 940, 950, 1030, 1035 shown in and described in relation to Figs. 9A-9B and 11C-11E. In some configurations, the fixation strip 1502 may include a wound sealing layer, which can be the same as or similar to the wound sealing layer 930, 1020 shown in and described in relation to Figs. 9A-9B and 11C-11F.

As shown in Fig. 12A, the sheet 1500 can include a plurality of fixation strips 1502. The sheet 1500 can include, for example, six fixation strips 1502. In some configurations, the sheet 1500 can include less than or more than six fixation strips 1502 (e.g., two, three, four, five, seven, eight, or more strips). In some configurations, the sheet 1500 can include a sheet width 1514. The sheet width 1514 can be between about 100 mm and about 500 mm, 150 mm and about 450 mm, about 200 mm and about 400 mm, or about 250 mm and about 350 mm. In some configurations, the fixation strip 1502 can include a strip width 1516. The strip width 1516 can be between about 10 mm and about 100 mm, about 20 mm and about 90 mm, about 30 mm and about 80 mm, about 40 mm and about 70 mm, or about 50 mm and about 60 mm.

As shown in Fig. 12B, the fixation strip 1502 can include a plurality of layers 1504, 1506, 1508, 1510, 1512 that can have the same or different lengths and/or widths. For example, the top layer 1504 can have a length 1520 that can be between about 100 mm and about 500 mm, 150 mm and about 450 mm, about 200 mm and about 400 mm, or about 250 mm and about 350 mm. The adhering layer 1506 can have a length 1518 that can be between about 100 mm and about 500 mm, 150 mm and about 450 mm, about 200 mm and about 400 mm, or about 250 mm and about 350 mm.

In some configurations, the one or more protective liners 1508, 1510, 1512 can include three protective liners 1508, 1510, 1512. In some configurations, the one or more protective liners can include less than or more than three protective liners (e.g., one, two, four, five, six, seven or more liners). The three protective liners 1508, 1510, 1512 can include two outer protective liners 1508, 1510 and a central protective liner 1512. At least one of the two outer protective liners 1508, 1510 can include an outer edge that can extend beyond the adhering layer 1506. For example, a distance 1524 between an outer edge of the adhering layer 1506 and the outer edge of at least one of the outer protective liners 1508, 1510 can be between about 1 mm and about 30 mm, about 5 mm and about 25 mm, or about 10 mm and about 20 mm. In some configurations, at least one of the outer edges of the outer protective liners 1508, 1510 can substantially align with an outer edge of the top layer 1504.

In some configurations, at least one of the outer protective liners 1508, 1510 can include a folded handle. The folded handle can be positioned at or near an inner edge of the outer protective liner 1508, 1510. The inner edge of the outer protective liner 1508, 1510 can be opposite the outer edge of the outer protective liner 1508, 1510. The folded handle can include a length 1522 that can be between about 10 mm and about 50 mm, about 15 mm and about 45 mm, about 20 mm and about 40 mm, or about 25 mm and about 35 mm. In some configurations, the folded handles may be spaced apart such that the outer protective liners 1508, 1510 do not cover the entire length of the adhering layer 1506. In some configurations, the central protective liner 1512 can be positioned over at least one of the folded handles. For example, the central protective liner 1512 can overlap the folded handles such that at least one of the outer edges of the central protective liner 1512 extends beyond at least one of the outer edges of the folded handles. A distance 1526 between an outer edge of the central protective liner 1512 and an outer edge of a folded handled can be between about 1 mm and about 20 mm, or about 5 mm and about 15 mm.

As shown in Fig. 12A, the plurality of fixation strips 1502 can be removably attached to one another. For example, the sheet 1500 can include a perforated portion 1528 between each of the plurality of fixation strips 1502 such that a user can tear along the perforated portion 1528 to remove a fixation strip 1502 from the sheet 1500. The perforated portion 1528 can extend through each of the layers 1504, 1506, 1508, 1510, 1512 of the fixation strips 1502. In some configurations, the perforated portion 1528 can include a continuous cut 1528a along the middle of the perforated portion 1528 and two perforated sections 1528b, 1528c on either side of the cut 1528a. For example, the continuous cut 1528a may have a length of between about 50 mm and about 350 mm, about 100 mm and about 300 mm, or about 150 mm and about 250 mm. In some configurations, the continuous cut 1528a can have a length that is greater than or equal to a length of the central protective liner 1512. The two perforated sections 1528b, 1528c can include a plurality of perforations that are each separated by an attached or bridging portion that connects adjacent strips 1502. The length of one or both of the two perforated sections can be between about 10 mm and about 100 mm, about 20 mm and about 90 mm, about 30 mm and about 80 mm, about 40 mm and about 70 mm, or about 50 mm and about 60 mm. As shown in Fig. 12C, the attached or bridging portions can include a length 1530 and the perforations can include a length 1532. The length 1530 of each of the attached or bridging portions can be between about 0.1 mm and about 2.0 mm, about 0.5 mm and about 1.5 mm, or about 0.5 mm. The length 1532 of each of the perforations can be between about 1.0 mm and about 4.0 mm, about 1.5 mm and about 3.5 mm, about 2.0 mm and about 3.0 mm, or about 2.5 mm. Advantageously, if a user removes the central protective liner 1512 without first separating adjacent fixation strips 1502, then only that central protective liner 1512 will be removed instead of each central protective liners 1512 of all of the fixation strips 1502.

To utilize the fixation strips 1502, a dressing may be applied to a wound site. A single fixation strip 1502 can be removed from the sheet 1500 by tearing along the perforated portion of the sheet 1500. The central protective liner 1512 may be removed. The outer protective liners 1508, 1510 may be removed by using the folded handles for exposing an adhesive surface of the adhering layer 1506. The adhesive surface may be applied to skin of a user and/or a dressing or any desired location. After sealing the fixation strip 1502 along an edge of a dressing and/or the skin of the user, the top layer 1504 may be removed from the adhering layer 1506. This may be repeated with as many fixation strips 1502 as are needed.

### Multilayer Drape Optionally Configured with Handles

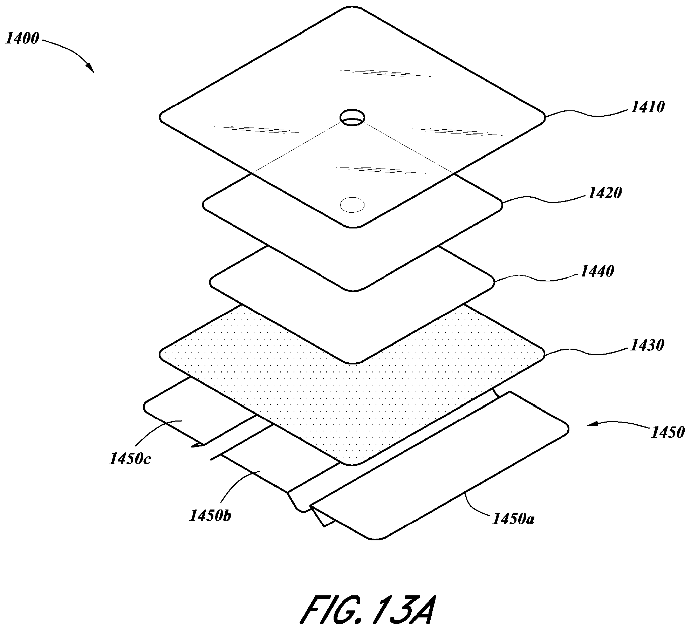

With reference to Figs. 13A-13B, configurations of a wound dressing or drape 1400, 1400' is shown. The wound dressing or drape 1400, 1400' can be used with any of the system configurations disclosed herein this section or elsewhere in the specification, such as in Figs 1-6. For example, such a drape could be incorporated as drape 5531 described above. Any component or step disclosed in any configuration in this specification can be used in any other configuration.

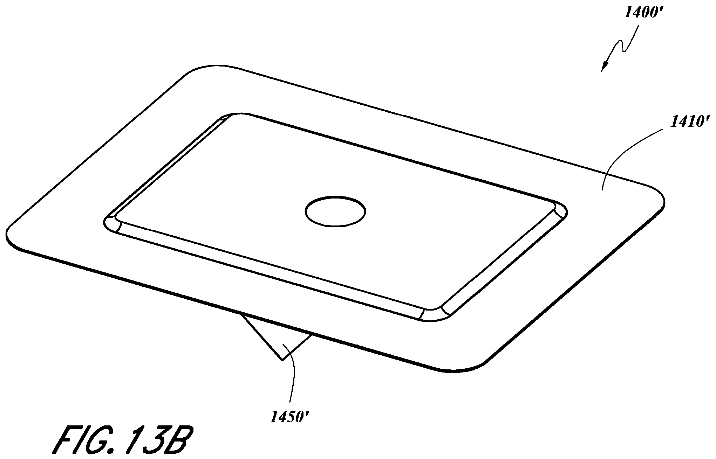

Fig. 13A shows a configuration of a drape 1400 comprising a square-like shape. In other configurations, the drape 1400 can include any of a number of shapes, such as a rectangular, an oval, a circle, or any geometric or non-geometric shape. For example, Fig. 13B illustrates a drape 1400' with a rectangular shape that can be the same as or similar to the drape 1400 shown in Fig. 13B. The drape 1400 can include four layers and at least one protective layer 1450. For example, the drape 1400 may include a top layer 1410, an intermediate layer 1420 (also referred to herein as a protective construction layer or PCL), a transmission layer 1440, and a wound contact layer 1430. The top layer 1410 can be positioned over the intermediate layer 1420 with the transmission layer 1440 being positioned between the intermediate layer 1420 and the wound contact layer 1430. Although Fig. 13A show the drape 1400 with four layers and a certain positioning of the layers, the drape 1400 can include less than or more than four layers and the layers can be arranged in a different order.

The top layer 1410 of the drape 1400 can be formed of a breathable film that allows for fluid evaporation, for example polyurethane. The film can be transparent, such that from the top view of the dressing 1400, other layer(s) underneath the top layer 1410 are also visible. The top layer 1410 can include an adhesive for securing the top layer 1410 to the intermediate layer 1420 and the wound contact layer 1430. Additionally, or alternatively, the adhesive of the top layer 1410 can secure the dressing 1400 to the periwound area, as described above. For example, the top layer 1410 can include an acrylic adhesive on a lower surface (i.e., the surface facing the other layers 1420, 1430, 1440) of the top layer 1410.

In some configurations, the top layer 1410 can include a width and a length. The width can be approximately 220 mm, approximately 270 mm, or approximately 420 mm. The width can be between approximately 150 mm to approximately 500 mm, approximately 200 mm to approximately 450 mm, approximately 250 mm to approximately 400 mm, approximately 300 mm to approximately 350 mm, or any suitable width. The length can be approximately 170 mm or approximately 270 mm. The length can be between approximately 100 mm to approximately 300 mm, approximately 125 mm to approximately 275 mm, approximately 150 mm to approximately 250 mm, approximately 175 mm to approximately 225 mm, or any suitable length.

In some configurations, the top layer 1410 can include a greater width and a greater length than the other layers 1420, 1430, 1440 such that the top layer 1410 includes a top border (not shown) that extends beyond the perimeter of the other layers 1420, 1430, 1440. In this configuration, the top border can adhere to the periwound area in use. In some configurations, the top layer 1410 and the wound contact layer 1430 can have the same length and width such that the top layer 1410 adheres to the wound contact layer 1430 to sandwich the other layer 1420, 1440 between the two layers 1410, 1430. In this configuration, the wound contact layer 1430 can adhere to the wound site and the periwound area in use. The top border can include a width that can be measured from an edge of the PCL 1420 to an edge of the top layer 1410. The width can be approximately 30 mm. The width can be between approximately 10 mm to approximately 50 mm, approximately 15 mm to approximately 45 mm, approximately 20 mm to approximately 40 mm, approximately 25 mm to approximately 35 mm, or any suitable width.

The size of the top layer 1410 can correspond with a size of the dressing, such as small, medium or large. The table below shows example dimensions of the top layer 1410 that can correspond with the small, medium or large sizes.

| **Size** | **Top Border width** | **Length L₂** | **Width W₂** |
|---|---|---|---|
| Small | 30 mm | 170 mm | 220 mm |
| Medium | 30 mm | 270 mm | 270 mm |
| Large | 30 mm | 270 mm | 420 mm |

The intermediate layer 1420 can be a protective construction layer (PCL) 1420 configured to provide support to the dressing 1400 and protect the top layer from the transmission layer 1440 (e.g., from being pierced by monofilaments of the transmission layer 1440 when the transmission layer comprises monofilaments). For example, the PCL 1420 can provide a layer of support between the top layer 1410 and the transmission layer 1440. The PCL 1420 can be configured to act as a masking or obscuring layer to help reduce the unsightly appearance of a drape 1400 during use due to the absorption of wound exudate. The PCL 1420 may be a colored portion of the transmission layer 1440, or may be a separate layer that covers the transmission layer 1420. The PCL 1420 may be one of a variety of colors such as blue, orange, yellow, green, or any color suitable for masking the presence of wound exudate in the drape 1400. For example, a blue PCL 1420 may be a shade of blue similar to the shade of blue commonly used for the material of medical gowns, scrubs, and drapes. Some configurations of the PCL 1420 may comprise polypropylene spunbond material. For example, the PCL 1420 can be manufactured from or comprise Glatfelter Vizorb^{®} 3055 MBAL90, Daltex^{®} REPEL, and/or a sterilisable thermally bonded nonwoven viscose and polypropylene non-woven fabric (e.g., Freudenberg M1556N, Vilene). Further, some configurations of the PCL 1420 may comprise a hydrophobic additive or coating. Some configurations may comprise a thin fibrous sheet of 60, 70, or 80 gsm.

The PCL 1420 may comprise at least one viewing window configured to allow a visual determination of the saturation level of the transmission layer 1440. The at least one viewing window may comprise at least one aperture made through the PCL 1420. The at least one viewing window may comprise at least one uncolored region of the PCL 1420. Some configurations of the PCL 1420 may comprise a plurality of viewing windows or an array of viewing windows.

The masking capabilities of the PCL 1420 can be partial or less than 100% obscuring to thereby allow clinicians to access the information they require by observing the spread of exudate across the dressing surface. The masking capabilities of the PCL 1420 may be partial due to material properties allowing wound exudate to slightly alter the appearance of the dressing 1400 or due to the presence of at least one viewing window in a completely obscuring material. The partial masking nature of the PCL 1420 may enable a skilled clinician to perceive a different colour caused by exudate, blood, by-products etc. in the dressing allowing for a visual assessment and monitoring of the extent of spread across the dressing. However, since the change in colour of the dressing from its clean state to a state with exudate contained may only be a slight change, the patient is unlikely to notice any aesthetic difference. Reducing or eliminating a visual indicator of wound exudate from a patient is likely to have a positive effect on their health, reducing stress for example.

The PCL 1420 can include a width and a length. For example, the length and width of the PCL 1420 can be less than the width and length of the top layer 1410, as described above. The width of the PCL 1420 can be approximately 160 mm, approximately 210 mm, or approximately 360 mm. The width can be between approximately 100 mm to approximately 400 mm, approximately 125 mm to approximately 375 mm, approximately 150 mm to approximately 350 mm, approximately 200 mm to approximately 325 mm, approximately 225 mm to approximately 300 mm, approximately 250 mm to approximately 300 mm, or any suitable width. The length of the PCL 1420 can be approximately 110 mm or approximately 210 mm. The length can be between approximately 50 mm to approximately 300 mm, approximately 75 mm to approximately 275 mm, approximately 100 mm to approximately 250 mm, approximately 125 mm to approximately 225 mm, approximately 150 mm to approximately 200 mm, or any suitable length.

In some configurations, the PCL 1420 can include a greater width and a greater length than the transmission layer 1440 such that the PCL 1420 includes a PCL border 1420a that extends beyond the perimeter of the transmission layer 1440. The PCL border 1420a can include a width that can be measured from an edge of the transmission layer 1440 to an edge of the PCL 1420. The width can be approximately 5 mm. The width can be between approximately 5 mm to approximately 20 mm, or any suitable width.

The size of the PCL 1420 can correspond with a size of the dressing, such as small, medium or large. The table below shows example dimensions of the PCL 1420 that can correspond with the small, medium or large sizes.

| **Size** | **PCL border 1420a width** | **PCL 1420 Length** | **PCL 1420 Width** |
|---|---|---|---|
| Small | 5 mm | 110 mm | 160 mm |
| Medium | 5 mm | 210 mm | 210 mm |
| Large | 5 mm | 210 mm | 360 mm |

The transmission layer 1440 can be configured to act as a transmission layer. In some embodiments, the transmission layer 1440 can be a porous material. The transmission layer 1440 may allow transmission of fluid, including liquid and gas, away from a wound site into upper layers of the drape 1400. In particular, the transmission layer 1440 can assist in distributing negative pressure evenly to the wound and periwound areas. This layer 1440 may be formed of a material having a three-dimensional structure, for example, a knitted or woven spacer fabric (for example Baltex 7970 weft knitted polyester) or a non-woven fabric could be used. The three-dimensional material can comprise a 3D spacer fabric material similar to the material described in International Publication WO 2013/175306 A2 and International Publication WO2014/020440. The three-dimensional material may also be the same or similar to the materials described above for upper and lower channel layers 5512 and 5516 and may be comprised of a fabric, for example a knitted or woven spacer fabric (such as a knitted polyester 3D fabric, Baltex 7970.RTM., or Gehring 879.RTM.) or a nonwoven material. Suitable materials may also include terry-woven or loop-pile materials. The fibers may not necessarily be woven, and can include felted and flocked (including materials such as Flotex.RTM.) fibrous materials.

Some configurations of the transmission layer 1440 may additionally or alternatively comprise a wicking or acquisition distribution layer (ADL). The ADL is another type of transmission layer that may be provided for communicating fluid through the drape 1400. The ADL may be configured to horizontally wick fluid such as wound exudate as it is absorbed upward through the layers of the drape 1400. Lateral wicking of fluid may advantageously increase moisture vapor permeation and efficient delivery of negative pressure to the wound site. Some embodiments of the ADL may comprise viscose, polyester, polypropylene, polyethylene, cellulose (for example polysaccharide or repeated disaccharide), or a combination of some or all of these, and the material may be needle-punched. Some configurations of the ADL may comprise polyethylene in the range of 40-150 grams per square meter (gsm). Some configurations of the ADL may comprise a heavy fibrous melt material. Some configurations of the ADL may be relatively porous to allow for the passage of fluids, including gas, therethrough. One example of an ADL may comprise a lightweight, felt-like, viscose material, which may be 80 gsm (or approximately 80 gsm). Some embodiments of the ADL may comprise cellulose in the range of 40-160 gsm (or about 40 to about 160 gsm), for example 80 (or about 80) gsm. The ADL may be constructed from a material which resists compression under the levels of negative pressure commonly applied during negative pressure therapy. The ADL may be constructed so as to advantageously vertically wick fluid, such as wound exudate. Facilitating rapid movement of wound exudate from the transmission layer to a port on, for example, the top layer 1410 is desirable. Additionally judicious choice of material can reduce re- wetting of liquid from the upper layers down into lower layers, this phenomenon is known as "back wetting" or "re-wetting". Suitable materials that show an enhancement of this effect include Slimcore TL4 (150 gsm) from Libeltex BVBA or equivalent.

Some configurations of the ADL may include several internal layers. For example, one material suitable for use as an ADL includes a lower wicking or acquisition layer comprising substantially vertically extending fibers for vertical wicking of fluid and further includes an upper distribution layer comprising substantially horizontally extending fibers for horizontal wicking of fluid. Some ADL materials can include three or more layers, for example a lower wicking layer and two upper distribution layers. Other configurations can have one or more distribution layers positioned between upper and lower acquisition layers.

In some embodiments, the transmission layer may additionally or alternatively comprise an absorbent material, and in other embodiments, the transmission layer may not have any absorbent material. If absorbent material is utilized, the absorbent material may be a foam or non-woven natural or synthetic material and which may optionally include or be super-absorbent material forms a reservoir for fluid, particularly liquid, removed from the wound site. The material of the transmission layer 1440 also prevents liquid collected in the drape 1400 from flowing in a sloshing manner. The transmission layer 1440 can help distribute fluid throughout the layer 1440 via a wicking action so that fluid is drawn from the wound site and stored throughout the transmission layer 1440. This can help prevent agglomeration in areas of the transmission layer 1440. The capacity of the absorbent material must be sufficient to manage the exudates flow rate of a wound when negative pressure is applied. Since in use the transmission layer 1440 experiences negative pressures the material of the transmission layer 1440 is chosen to absorb liquid under such circumstances. A number of materials exist that are able to absorb liquid when under negative pressure, for example superabsorber material. In addition to materials described above, the transmission layer 1440 may be manufactured from or comprise ALLEVYN^{™} foam, Freudenberg 114-224-4 and/or Chem-Posite^{™} 11C-450.

In some configurations, the transmission layer 1440 can include non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers may lead to strong capillary action in the fibrous pad which helps distribute liquid. In this way, the super-absorbent material is efficiently supplied with liquid. Also, all regions of the transmission layer 1440 are provided with liquid. The wicking action also assists in bringing liquid into contact with the top layer 1410 to aid increase transpiration rates of the drape 1400. The wicking action may also assist in delivering liquid downwards towards the wound bed when exudation slows or halts. This delivery process can help maintain the transmission layer 1440 and lower wound bed region in a moist state which helps prevent crusting within the drape 1400, which could lead to blockages, and helps maintain an environment optimized for wound healing.

In some configurations, the transmission layer 1440 may include an air-laid material or any suitable material disclosed herein. Heat fusible fibers may optionally be used to assist in holding the structure of the drape 1400 together. It will be appreciated that rather than using super-absorbing particles or in addition to such use, super-absorbing fibers may be utilized according to certain configurations of the present disclosure. An example of a suitable material is the Product Chem-Posite^{™} 11 C available from Emerging Technologies Inc (ETi) in the USA.

Optionally, according to certain configurations of the present disclosure, the transmission layer 1440 may include synthetic stable fibers and/or bi-component stable fibers and/or natural stable fibers and/or super-absorbent fibers. Fibers in the transmission layer 1440 may be secured together by latex bonding or thermal bonding or hydrogen bonding or a combination of any bonding technique or other securing mechanism. In some configurations, the transmission layer 1440 may be formed by fibers which operate to lock super-absorbent particles within the transmission layer 1440. Such an arrangement can help ensure that super-absorbent particles do not move external to the transmission layer 1440 and towards an underlying wound bed. This may improve functionality because when negative pressure is applied there is a tendency for the transmission layer 1440 to collapse downwards and this action may push super-absorbent particle matter into a direction towards the wound bed if they were not locked away by the fibrous structure of the transmission layer 1440.

The transmission layer 1440 may comprise multiple fibers. The fibers can be strand-like and made from cellulose, polyester, viscose or the like. Dry absorbent particles can be distributed throughout the transmission layer 1440 ready for use. In some configurations, the transmission layer 1440 includes a pad of cellulose fibers and a plurality of super absorbent particles. In additional configurations, the transmission layer 1040 can be a non-woven layer of randomly orientated cellulose fibers.

The transmission layer 1440 may include a length and a width. The width of the transmission layer 1440 can be approximately 150 mm, approximately 200 mm, or approximately 350 mm. The width can be between approximately 100 mm to approximately 400 mm, approximately 150 mm to approximately 350 mm, approximately 200 mm to approximately 300 mm, or any suitable width. The length of the transmission layer 1440 can be approximately 100 mm or approximately 200 mm. The length can be between approximately 50 mm to approximately 300 mm, approximately 75 mm to approximately 275 mm, approximately 100 mm to approximately 250 mm, approximately 125 mm to approximately 225 mm, approximately 150 mm to approximately 200 mm, or any suitable length.

The size of the transmission layer 1440 can correspond with a size of the dressing, such as small, medium or large. The table below shows example dimensions of the transmission layer 1440 that can correspond with the small, medium or large sizes.

| **Size** | **Transmission layer 1440 Length** | **Transmission layer 1440 Width** |
|---|---|---|
| Small | 110 mm | 160 mm |
| Medium | 210 mm | 210 mm |
| Large | 210 mm | 360 mm |

The wound contact layer 1430 may include a length and a width. The width of the wound contact layer 1430 can be approximately 220 mm, approximately 270 mm, or approximately 420 mm. The width can be between approximately 200 mm to approximately 500 mm, approximately 250 mm to approximately 450 mm, approximately 300 mm to approximately 400 mm, or any suitable width. The length of the wound contact layer 1430 can be approximately 170 mm or approximately 270 mm. The length can be between approximately 100 mm to approximately 300 mm, approximately 125 mm to approximately 275 mm, approximately 150 mm to approximately 250 mm, approximately 175 mm to approximately 225 mm, or any suitable length.

In some configurations, the wound contact layer 1430 can be the same size as the top layer 1410. In this configurations, the wound contact layer 1430 can adhere to the wound site and the periwound area in use. For example, the wound contact layer 1430 can be the same as or similar to any of the wound contact layers described herein. The wound contact layer 1430 may include a wound contact border (not shown). The wound contact border may be the same size or a different size as the top border. The wound contact border can include a width that can be measured from an edge of the PCL 1420 to an edge of the wound contact layer 1430. The width can be approximately 30 mm. The width can be between approximately 10 mm to approximately 50 mm, approximately 15 mm to approximately 45 mm, approximately 20 mm to approximately 40 mm, approximately 25 mm to approximately 35 mm, or any suitable width.

The size of the wound contact layer 1430 can correspond with a size of the dressing, such as small, medium or large. The table below shows example dimensions of the wound contact layer 1430 that can correspond with the small, medium or large sizes.

| **Size** | **Wound Contact Border width** | **Wound Contact Layer 1430 Length** | **Wound Contact Layer 1430 Width** |
|---|---|---|---|
| Small | 30 mm | 170 mm | 220 mm |
| Medium | 30 mm | 270 mm | 270 mm |
| Large | 30 mm | 270 mm | 420 mm |

As shown in Figs. 13A and 13B, the layers 1410, 1420, 1430, 1440 may overlap with a periwound area to allow for NPWT to that area. For example, the wound contact layer 1430 may be positioned on a wound site and the transmission layer 1440 can be positioned over the wound contact layer 1430. The wound contact layer 1430 can adhere to the periwound area surrounding the wound site. The PCL 1420 can be positioned over the transmission layer 1440 such that the PCL 1420 can reduce the risk of the material from the transmission layer 1440 piercing the top layer 1410. The top layer 1410 can be positioned above the PCL 1420 such that the portions of the top layer 1410 that extend beyond the PCL 1420 and the transmission layer 1440 can adhere to the wound contact layer 1430 and/or the periwound area. When these overlapping layers 1410, 1420, 1430, 1440 are placed on a periwound area, negative pressure can be applied to the top layer 1410 and through the other layer 1420, 1430, 1440 to the periwound area, which is described in further detail below. An opening or aperture may be provided in the top layer 1410 and in the PCL 1420, such as in a center of these layers, to provide negative pressure to the wound site and to the periwound area through the drape, such as through a port or suction adapter as described in this specification.

The drape 1400 may include at least one protective release layer 1450. The at least one protective release layer 1450 may comprise a single integral component or several overlapping components. The illustrated configuration in Fig. 13A has three overlapping components: a center release strip 1450b and first and second side release strips 1450a, 1450c. Although three release strips are shown, the drape 1400 can include more or less than three release strips (e.g., one, two, four, five, six, or more release strips). The at least one protective release layer 1450 may be removable before use. For example, each of the release strips 1450a, 1450b, 1450c may include a handle and/or a pair of handles, which can be configured to remove the strips when actuated 1450a, 1450b, 1450c. A user, for example, can pull on the handle(s) to remove the strips 1450a, 1450b, 1450c. The at least one protective release layer 1450 may be removably attached to the lower surface of the wound contact layer 1430 and/or the top layer 1410 to maintain the adhesiveness of the silicone adhesive of the wound contact layer 1430 and/or the acrylic adhesive of the top layer 1410. As shown in Fig. 12A, the first and second release strips 1450a, 1450c can be positioned on opposite sides of the center release strips 1450b. The first and second release strips 1450a, 1450c can overlap opposite sides of the center release strips 1450b.

### Method of Treating a Wound with a Multilayer Drape

Some embodiments described herein provide a method of treating a wound using wound dressings and drapes as described herein, such as the wound dressing or drape 800described above in relation to Figs 13A-13B. The method of treating a wound may comprise removing the center release strip 1450b from the drape 1400. The drape 1400 can be positioned on the wound such that the wound contact layer 1430 contacts the wound and/or a periwound area surrounding the wound. The wound contact layer 1430 can be adhered to the wound and/or the periowound area. The drape 1400 can optionally be repositioned over the wound and periwound area until the drape 1400 is in a proper position to provide effective treatment to the wound. The second side release strips 1450a, 1450c can be removed from the adhesive surface of the top layer 1410. The portions of the top layer 1410 that extend over the wound contact layer 1430 can be adhered to the periwound area such that the drape 1400 is secured to the patient and negative pressure can be applied to the wound and periwound area through the drape 1400. Optionally, one or more fixation strips 900, 1000 can be applied to the periphery of the drape 1400.

Some embodiments described herein provide a method of treating a wound using wound dressings and drapes as described herein, such as the drape 1400 described above in relation to Figs 13A-13B. The method of treating a wound may comprise removing the at least one protective release layer 1450. The drape 1400 can be positioned on the wound such that the wound contact layer 1430 contacts the wound and/or a periwound area surrounding the wound. The wound contact layer 1430 can be adhered to the wound and/or the periowound area. The drape 1400 can optionally be repositioned over the wound and periwound area until the drape 1400 is in a proper position to provide effective treatment to the wound. A port or suction adapter as described elsewhere in this specification may be utilized to supply negative pressure to the drape 1400. The port or suction adapter may be pre-applied to the drape over the opening in the top layer 1410 before the drape is positioned on the wound, or it may be applied after the drape is positioned on the wound.

In some alternative configurations, the methods of treating a wound may comprise using the drape 1400 under ambient conditions not in connection with a negative pressure wound therapy system as described above, or described elsewhere herein.

### Terminology

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. Furthermore, the features and attributes of the specific embodiments disclosed above may be combined in different ways to form additional embodiments, all of which fall within the scope of the present disclosure.

Although the present disclosure includes certain embodiments, examples and applications, it will be understood by those skilled in the art that the present disclosure extends beyond the specifically disclosed embodiments to other alternative embodiments or uses and obvious modifications and equivalents thereof, including embodiments which do not provide all of the features and advantages set forth herein.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Likewise the term "and/or" in reference to a list of two or more items, covers all of the following interpretations of the word: any one of the items in the list, all of the items in the list, and any combination of the items in the list. Further, the term "each," as used herein, in addition to having its ordinary meaning, can mean any subset of a set of elements to which the term "each" is applied. Additionally, the words "herein," "above," "below," and words of similar import, when used in this application, refer to this application as a whole and not to any particular portions of this application.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

## Claims

1. An apparatus for sealing a wound site, the apparatus comprising:
a fixation strip comprising:
a wound sealing layer comprising a first adhesive surface, the first adhesive surface comprising a first adhesive;
an adhering layer overlying the wound sealing layer and extending outward beyond the wound sealing layer, the adhering layer comprising a second adhesive surface comprising a second adhesive;
at least one protective liner removably adhered to the first adhesive surface of the wound sealing layer; and
wherein the fixation strip is configured for placement over an edge of a wound dressing such that, in use, the wound sealing layer is placed over the wound or the area surrounding the wound and the adhering layer is placed over the wound dressing.

2. The apparatus of Claim 1, wherein the at least one protective liner is removably adhered to the first adhesive surface of the wound sealing layer and the second adhesive surface of the adhering layer.

3. The apparatus of claim 1 or claim 2, wherein the fixation strip comprises a width and a length, the second adhesive extending along the width of the fixation strip.

4. The apparatus of Claim 3, wherein the first adhesive extends along a portion of the width of the fixation strip.

5. The apparatus of Claim 4, wherein the at least one protective liner further comprises a first protective liner and a second protective liner, and wherein the first protective liner is removably adhered to the first adhesive surface of the wound sealing layer and the second protective liner is removably adhered to the second adhesive surface of the adhering layer.

6. The apparatus of Claim 5, wherein the first protective liner comprises a release handle configured to remove the first protective liner from the wound sealing layer when actuated, and wherein the second protective liner comprises a release handle configured to remove the second protective liner from the adhering layer when actuated.

7. The apparatus of Claim **1,** wherein the at least one protective liner further comprises a first outer protective liner, a second outer protective liner and a central protective liner.

8. The apparatus of Claim 7, wherein the first outer protective liner is removably adhered to an outer edge portion of the first adhesive surface of the wound sealing layer, wherein the second outer protective liner is removably adhered to a portion of the second adhesive surface of the adhering layer extending outward beyond the wound sealing layer, and wherein the central protective liner is removably adhered to the first adhesive surface of the wound sealing layer between the first and second outer protective liners.

9. The apparatus of Claim 7 or 8, wherein at least one of the first outer protective liner, the second outer protective liner, and the central protective liner comprises a release handle configured to remove the respective liner from the wound sealing layer and/or the adhering layer when actuated.

10. The apparatus of Claim 7, wherein the first outer protective liner, the second outer protective liner, and the central protective liner each comprise a width that extends a width of the fixation strip to removably adhere to the first adhesive surface of the wound sealing layer and a portion of the second adhesive surface of the adhering layer that extends outward beyond the wound sealing layer.

11. The apparatus of any one of the preceding claims, further comprising a carrier layer removably adhered to a non-adhesive surface of the adhering layer.

12. The apparatus of any one of the preceding claims, wherein the first adhesive comprises a silicone adhesive configured to adhere to the wound site or to skin surrounding the wound site.

13. The apparatus of any one of the preceding claims, wherein the second adhesive comprises an acrylic adhesive configured to adhere to a wound dressing.

14. The apparatus of any one of the previous claims, wherein the wound sealing layer comprises a flexible polymer.

15. The apparatus of any one of the previous claims, wherein the adhering layer comprises a flexible film.

16. The apparatus of any one of the previous claims, further comprising a wound dressing including the fixation strip of any one of claims 1 to 15 applied thereto.

17. The apparatus of claim 16, wherein the adhering layer includes an extended side and wherein the extended side is adhered to the upper surface of the wound dressing.

## Patentansprüche

1. Vorrichtung zum Versiegeln einer Wundstelle, wobei die Vorrichtung umfasst:
einen Befestigungsstreifen, umfassend:
eine Wundversiegelungsschicht, die eine erste Klebeoberfläche umfasst, wobei die erste Klebeoberfläche einen ersten Klebstoff umfasst;
eine Haftschicht, die über der Wundversiegelungsschicht liegt und sich nach außen über die Wundversiegelungsschicht hinaus erstreckt, wobei die Haftschicht eine zweite Klebeoberfläche umfasst, die einen zweiten Klebstoff umfasst;
mindestens eine abnehmbar an der ersten Klebeoberfläche der Wundversiegelungsschicht haftende Schutzdeckschicht; und
wobei der Befestigungsstreifen so ausgestaltet ist, dass er über einem Rand einer Wundauflage platziert wird, so dass bei Gebrauch die Wundversiegelungsschicht über der Wunde oder dem die Wunde umgebenden Bereich platziert wird, und die Haftschicht über der Wundauflage platziert wird.

2. Vorrichtung nach Anspruch 1, wobei die mindestens eine Schutzdeckschicht abnehmbar an der ersten Klebeoberfläche der Wundversiegelungsschicht und der zweiten Klebeoberfläche der Haftschicht haftet.

3. Vorrichtung nach Anspruch 1 oder Anspruch 2, wobei der Befestigungsstreifen eine Breite und eine Länge aufweist, wobei sich der zweite Klebstoff entlang der Breite des Befestigungsstreifens erstreckt.

4. Vorrichtung nach Anspruch 3, wobei sich der erste Klebstoff entlang eines Abschnitts der Breite des Befestigungsstreifens erstreckt.

5. Vorrichtung nach Anspruch 4, wobei die mindestens eine Schutzdeckschicht ferner eine erste Schutzdeckschicht und eine zweite Schutzdeckschicht umfasst, und wobei die erste Schutzdeckschicht abnehmbar an der ersten Klebeoberfläche der Wundversiegelungsschicht haftet und die zweite Schutzdeckschicht abnehmbar an der zweiten Klebeoberfläche der Haftschicht haftet.

6. Vorrichtung nach Anspruch 5, wobei die erste Schutzdeckschicht einen Abnehmgriff umfasst, der ausgestaltet ist, um bei Betätigung die erste Schutzdeckschicht von der Wundversiegelungsschicht zu entfernen, und wobei die zweite Schutzdeckschicht einen Abnehmgriff umfasst, der ausgestaltet ist, um bei Betätigung die zweite Schutzdeckschicht von der Haftschicht zu entfernen.

7. Vorrichtung nach Anspruch 1, wobei die mindestens eine Schutzdeckschicht ferner eine erste äußere Schutzdeckschicht, eine zweite äußere Schutzdeckschicht und eine zentrale Schutzdeckschicht umfasst.

8. Vorrichtung nach Anspruch 7, wobei die erste äußere Schutzdeckschicht abnehmbar an einem äußeren Randabschnitt der ersten Klebeoberfläche der Wundversiegelungsschicht haftet, wobei die zweite äußere Schutzdeckschicht abnehmbar an einem Abschnitt der zweiten Klebeoberfläche der Haftschicht haftet, der sich nach außen über die Wundversiegelungsschicht hinaus erstreckt, und wobei die zentrale Schutzdeckschicht zwischen der ersten und zweiten äußeren Schutzdeckschicht abnehmbar an der ersten Klebeoberfläche der Wundversiegelungsschicht haftet.

9. Vorrichtung nach Anspruch 7 oder 8, wobei mindestens eine der ersten äußeren Schutzdeckschicht, der zweiten äußeren Schutzdeckschicht und der zentralen Schutzdeckschicht einen Abnehmgriff aufweist, der ausgestaltet ist, um bei Betätigung die jeweilige Deckschicht von der Wundversiegelungsschicht und/oder der Haftschicht zu entfernen.

10. Vorrichtung nach Anspruch 7, wobei die erste äußere Schutzdeckschicht, die zweite äußere Schutzdeckschicht und die zentrale Schutzdeckschicht jeweils eine Breite aufweist, die sich über eine Breite des Befestigungsstreifens erstreckt, um abnehmbar an der ersten Klebeoberfläche der Wundversiegelungsschicht zu haften, und einen Abschnitt der zweiten Klebeoberfläche der Haftschicht, der sich nach außen über die Wundversiegelungsschicht hinaus erstreckt.

11. Vorrichtung nach einem der vorhergehenden Ansprüche, ferner umfassend eine Trägerschicht, die abnehmbar an einer Nicht-Klebeoberfläche der Haftschicht haftet.

12. Vorrichtung nach einem der vorhergehenden Ansprüche, wobei der erste Klebstoff einen Silikonklebstoff umfasst, der so ausgestaltet ist, dass er an der Wundstelle oder an der die Wundstelle umgebenden Haut haftet.

13. Vorrichtung nach einem der vorhergehenden Ansprüche, wobei der zweite Klebstoff einen Acrylklebstoff umfasst, der so ausgestaltet ist, dass er an einer Wundauflage haftet.

14. Vorrichtung nach einem der vorhergehenden Ansprüche, wobei die Wundversiegelungsschicht ein flexibles Polymer umfasst.

15. Vorrichtung nach einem der vorhergehenden Ansprüche, wobei die Haftschicht eine flexible Folie umfasst.

16. Vorrichtung nach einem der vorhergehenden Ansprüche, ferner umfassend eine Wundauflage einschließlich des Befestigungsstreifens nach einem der Ansprüche 1 bis 15, der darauf aufgebracht ist.

17. Vorrichtung nach Anspruch 16, wobei die Haftschicht eine verlängerte Seite umfasst, und wobei die verlängerte Seite an der oberen Oberfläche der Wundauflage haftet.

## Revendications

1. Appareil destiné à sceller le site d'une plaie, l'appareil comprenant :
une bande de fixation comprenant :
une couche de scellement de plaie comprenant une première surface adhésive, la première surface adhésive comprenant un premier adhésif ;
une couche adhésive recouvrant la couche de scellement de plaie et s'étendant vers l'extérieur au-delà de la couche de scellement de plaie, la couche adhésive comprenant une deuxième surface adhésive comprenant un deuxième adhésif ;
au moins une doublure protectrice collée de façon amovible à la première surface adhésive de la couche de scellement de plaie ; et
dans lequel la bande de fixation est conçue pour être placée au-dessus d'un bord d'un pansement de telle sorte que, à l'usage, la couche de scellement de plaie est placée au-dessus de la plaie ou de la zone entourant la plaie et la couche adhésive est placée au-dessus du pansement.

2. Appareil de la revendication 1, dans lequel l'au moins une doublure protectrice est collée de façon amovible à la première surface adhésive de la couche de scellement de plaie et à la deuxième surface adhésive de la couche adhésive.

3. Appareil de la revendication 1 ou la revendication 2, dans lequel la bande de fixation possède une largeur et une longueur, le deuxième adhésif s'étendant le long de la largeur de la bande de fixation.

4. Appareil de la revendication 3, dans lequel le premier adhésif s'étend le long d'une partie de la largeur de la bande de fixation.

5. Appareil de la revendication 4, dans lequel l'au moins une doublure protectrice comprend en outre une première doublure protectrice et une deuxième doublure protectrice, et dans lequel la première doublure protectrice est collée de façon amovible à la première surface adhésive de la couche de scellement de plaie et la deuxième doublure protectrice est collée de façon amovible à la deuxième surface adhésive de la couche adhésive.

6. Appareil de la revendication 5, dans lequel la première doublure protectrice comprend une poignée de libération conçue pour retirer la première doublure protectrice de la couche de scellement de plaie lorsqu'elle est actionnée, et dans lequel la deuxième doublure protectrice comprend une poignée de libération conçue pour retirer la deuxième doublure protectrice de la couche adhésive lorsqu'elle est actionnée.

7. Appareil de la revendication 1, dans lequel l'au moins une doublure protectrice comprend en outre une première doublure protectrice extérieure, une deuxième doublure protectrice extérieure et une doublure protectrice centrale.

8. Appareil de la revendication 7, dans lequel la première doublure protectrice extérieure est collée de façon amovible à une partie de bord extérieur de la première surface adhésive de la couche de scellement de plaie, dans lequel la deuxième doublure protectrice extérieure est collée de façon amovible à une partie de la deuxième surface adhésive de la couche adhésive s'étendant vers l'extérieur au-delà de la couche de scellement de plaie, et dans lequel la doublure protectrice centrale est collée de façon amovible à la première surface adhésive de la couche de scellement de plaie entre la première et la deuxième doublure protectrice extérieure.

9. Appareil de la revendication 7 ou 8, dans lequel au moins une doublure parmi la première doublure protectrice extérieure, la deuxième doublure protectrice extérieure et la doublure protectrice centrale comprend une poignée de libération conçue pour retirer la doublure respective de la couche de scellement de plaie et/ou de la couche adhésive lorsqu'elle est actionnée.

10. Appareil de la revendication 7, dans lequel la première doublure protectrice extérieure, la deuxième doublure protectrice extérieure et la doublure protectrice centrale possèdent chacune une largeur qui s'étend sur une largeur de la bande de fixation pour coller de façon amovible à la première surface adhésive de la couche de scellement de plaie et à une partie de la deuxième surface adhésive de la couche adhésive qui s'étend vers l'extérieur au-delà de la couche de scellement de plaie.

11. Appareil de l'une quelconque des revendications précédentes, comprenant en outre une couche de support collée de façon amovible à une surface non adhésive de la couche adhésive.

12. Appareil de l'une quelconque des revendications précédentes, dans lequel le premier adhésif comprend un adhésif à base de silicone conçu pour adhérer au site de la plaie ou à la peau entourant le site de la plaie.

13. Appareil de l'une quelconque des revendications précédentes, dans lequel le deuxième adhésif comprend un adhésif acrylique conçu pour adhérer à un pansement.

14. Appareil de l'une quelconque des revendications précédentes, dans lequel la couche de scellement de plaie comprend un polymère souple.

15. Appareil de l'une quelconque des revendications précédentes, dans lequel la couche adhésive comprend un film souple.

16. Appareil de l'une quelconque des revendications précédentes, comprenant en outre un pansement comportant la bande de fixation de l'une quelconque des revendications 1 à 15 appliquée à celui-ci.

17. Appareil de la revendication 16, dans lequel la couche adhésive comporte un côté étendu et dans lequel le côté étendu est collé à la surface supérieure du pansement.
